# EUROPÄISCHE PATENTSCHRIFT

(11) **EP 2 797 571 B1**
(45) Veröffentlichungstag und Bekanntmachung des Hinweises auf die Patenterteilung: **24.07.2019**
(21) Anmeldenummer: 12798317.9
(22) Anmeldetag: 11.12.2012
(51) Int. Cl.: A61K 8/36, A61Q 5/02, A61K 8/60, A61Q 5/12, A61Q 19/10

(54) **WÄSSRIGE HAAR- UND HAUTREINIGUNGSZUSAMMENSETZUNGEN, ENTHALTEND BIOTENSIDE**
AQUEOUS HAIR AND SKIN CLEANING COMPOSITIONS COMPRISING BIOTENSIDES
COMPOSITIONS AQUEUSES POUR LE NETTOYAGE DES CHEVEUX ET DE LA PEAU, CONTENANT DES TENSIOACTIFS BIOLOGIQUES

(30) Priorität: 28.12.2011 DE 102011090030
(43) Veröffentlichungstag der Anmeldung: 05.11.2014
(73) Patentinhaber: Evonik Degussa GmbH, 45128 Essen (DE)
(72) Erfinder: ALLEF, Petra, 45128 Essen (DE); HARTUNG, Christian, 45133 Essen (DE); SCHILLING, Martin, 53115 Bonn (DE)
(74) Vertreter: Evonik Patent Association
(86) Internationale Anmeldenummer: PCT/EP2012/075024
(87) Internationale Veröffentlichungsnummer: WO 2013/098066

(56) Entgegenhaltungen:
- WO-A1-2012/167815
- WO-A2-2004/108063
- JP-A- 2008 069 075
- JP-A- 2009 275 145
- JP-A- 2011 026 277
- JP-A- 2011 046 634
- S. KULKARNI ET AL.: "Production and isolation of biosurfactant-sophorolipid and its application in body wash formulation", ASIAN JR. OF MICROBIOL. BIOTECH. ENV. SC., Bd. 13, Nr. 1, 2011, Seiten 217-221, XP9178711,
- CLETUS P KURTZMAN ET AL: "Production of sophorolipid biosurfactants by multiple species of the Starmerella (Candida) bombicola yeast clade", FEMS MICROBIOLOGY LETTERS, WILEY-BLACKWELL PUBLISHING LTD, GB, vol. 311, no. 2, 20 October 2001 (2001-10-20), pages 140-146, XP002636233, ISSN: 0378-1097, DOI: 10.1111/J.1574-6968.2010.02082.X [retrieved on 2010-08-25]
- "International Cosmetic Ingredient Dictionary and Handbook", 2010, Personal Care Products Council vol. 3, pages 3461-3462,

## Beschreibung

Die vorliegende Erfindung ist gerichtet auf Zusammensetzungen, insbesondere Haar- und Hautreinigungszusammensetzungen, besonders bevorzugt Formulierungen zur Reinigung und Pflege von menschlichen oder tierischen Körperteilen, insbesondere Haut und Haaren bzw. Fell oder Federn. Die erfindungsgemäßen Zusammensetzungen enthalten ein oder mehrere Biotenside, ein oder mehrere Fettsäuren sowie Wasser. Die Zusammensetzungen können z. B. reinigende oder pflegende Formulierungen, wie z.B. Shampoos, Konditionierer, Duschgele, Körperreinigungsmittel oder Hautreinigungsmittel sein.

Es besteht seit langem der Bedarf milde Reinigungszusammensetzungen bereitzustellen, die insbesondere mild zu Haut und Haaren sind. Solche Reinigungszusammensetzungen sollen nicht nur mild zu Haut und Haaren sein, sondern auch über weitere wünschenswerte Eigenschaften verfügen, wie z.B. gute Lagerstabilität und gutes Schäumungsverhalten. Zudem besteht großer Bedarf an Tensiden, die komplett auf nachwachsenden Rohstoffen basieren und durch milde Bedingungen nachhaltig produziert werden können (z.B. durch Fermentation).

Es sind schon zahlreiche milde Tensidsysteme vorgestellt worden, mit denen versucht wurde, entsprechende Reinigungszusammensetzungen herzustellen. Häufig zeigten die milden Tensidsysteme ein geringeres Schaumvermögen, so dass eine größere Zugabe von Tensidsystem notwendig wurde, was wiederum zu einer geringeren Milde des Produkts führte.

Als geeignete Tensidsysteme, die zu milden Reinigungszusammensetzungen mit brauchbarem Schaumverhalten führen, sind solche vorgestellt worden, die Sulfosuccinate als Tenside in Kombination mit amphoteren Tensiden enthalten. Solche Systeme werden unter anderem in EP 1771148 und EP 1771538 beschrieben.

Nachteilig an Sulfosuccinat-Systemen war deren unvorhersehbares Verhalten in Bezug auf Viskositätseinstellung und Lagerstabilität, insbesondere deren Neigung zur Gelbildung. Gemäß EP 1771538 sind die Hydrolyseprodukte der Sulfosuccinate für die schlechte Lagerstabilität solcher Reinigungszusammensetzungen verantwortlich.

Im Stand der Technik ist die Verwendung von sogenannten Biotensiden zur Herstellung von Zusammensetzungen zur Reinigung von Haut und/oder Haaren bzw. als Haushaltsreiniger und Textilwaschmittel bekannt.

In US 7,985,722 werden Formulierungen, die Rhamnolipide aufweisen und deren Verwendung als Reinigungs-, Desinfektions- oder Deodorisierungsmittel beschrieben. In den Beispielen werden diese Formulierungen, die 5 % an Rohrhamonolipid und Wasser und Ethanol als Lösemittel/Träger aufweisen, z. B. als Shampoo/Reinigungsmittel für Haustiere eingesetzt. Für die Anwendung beim Menschen wurde eine 2%-ige Lösung von Rohrhamnolipid in Wasser/Ethanol verwendet. Das Rhamnolipid wurde zu einer Shampoo-Grundformulierung gegeben. In einem Konzentrationsbereich von 0,01 bis 35 % an konzentriertem Rohrhamnolipid wurde hier eine Rötung der Haut beobachtet (Beispiel 4).

In US 7,556,654 werden organische Reiniger beschrieben, die Biosurfactants, insbesondere Sophorolipide, und ein oder mehrere Enzyme aufweisen.

EP 0 499 434 beschreibt Mittel zum Waschen von Textilien, Geschirr und Flächen im Haushalt enthaltend ein oder mehrere Glykolipidbiotenside, insbesondere Sophorolipide, Glucoselipide, Cellobioselipiden, Trehaloselipiden und Rhamnolipide. Dabei wird in den Beispielen eine besonders gute Wasch-/Reinigungsleistung beschrieben, wenn Gemische von Glykolipidbiotensid, insbesondere Rhamnolipid, mit Nicht-Glykolipid-Biotensid, wie z. B. ethoxyliertem Dodecylalkohol oder Di-C8-Sulfosuccinat, verwendet werden. Das Nicht-Glykolipid-Biotensid liegt in der lamellaren Phase vor, das Glykolipid-Biotensid in der mizellaren Phase.

EP 1 445 302 beschreibt Reinigungsmittelzusammensetzungen, die mindestens ein Glykolipid-Biotensid und ein Nicht-Glykolipid-Biotensid aufweisen, wobei mindestens ein Nicht-Glykolipid-Biotensid in der mizellaren Phase vorliegt.

In EP 1 411 111 werden bioabbaubare niedrig-schäumende Detergenzzusammensetzungen beschrieben, die ein Gemisch von zwei Sophorolipiden sowie Detergenzhilfskomponenten, ausgewählt aus Enzym, Sauerstoff-Bleichmittel, Bleichaktivator, alkalischem Aufbaustoff, Sequestiermittel, Fluidwiederbildungsmittel und neutralem anorganischem Salz aufweisen.

Aus WO 03/006146 sind wässrige Zubereitungen, enthaltend oberflächenaktive Fermentationsprodukten und nichtionische Tenside sowie deren Verwendung zur Herstellung von kosmetischen Zubereitungen, bekannt.

Aus JP 2009 275145 A sind Reinigungszubereitungen enthaltend Sophorolipide bekannt.

Aufgabe der vorliegenden Erfindung war die Bereitstellung von Zusammensetzungen, insbesondere Haar- und Hautreinigungszusammensetzungen, besonders bevorzugt Formulierungen zur Reinigung und Pflege von menschlichen oder tierischen Körperteilen, insbesondere Haut und Haaren bzw. Fell oder Federn, die einen der mehrere der Nachteile der bekannten Formulierungen nicht aufweisen. Die erfindungsgemäßen Zusammensetzungen sollten vorzugsweise in möglichst großem Umfang biologisch abbaubar, gut verträglich, also insbesondere nur eine geringe bis gar keine Rötung von Haut und/oder Augen verursachen, eine gute Hautpflegeleistung aufweisen und/oder möglichst vollständig auf natürlichen Rohstoffen basieren.

Überrachenderweise wurde gefunden, dass Zusammensetzungen, wie in den Ansprüchen definiert und nachfolgend beschrieben, eine oder mehrere der genannten Aufgaben lösen.

Unerwarteterweise zeigte sich im Handwaschtest (HWT), dass die zusätzlich vorhandene Fettsäure (Ölsäure) einen positiven Effekt hat und wider Erwarten keinen signifikanten Auswirkungen auf Schaum oder Verdickungseigenschaften der Tensidlösung hat.

Gegenstand der vorliegenden Erfindung sind deshalb Zusammensetzungen enthaltend Wasser, mindestens ein Biotensid ausgewählt aus Rhamnolipiden und Sophorolipiden und mindestens eine Fettsäure ausgewählt aus Heptansäure, Octansäure, Pelargonsäure, Decansäure, Dodecansäure, Tetradecansäure, Palmitinsäure, Margarinsäure, Stearinsäure, Arachinsäure, Behensäure, Tetracosansäure, Cerotinsäure, Triacontansäure, Tubercolostearinsäure, Ölsäure, Elaidinsäure, Erucasäure, Linolsäure, Linolensäure, Elaeostearinsäure, Arachidonsäure, Eicosapentaensäure und Clupanodonsäure, welche dadurch gekennzeichnet sind, dass der Anteil der Summe aller Tenside an den Zusammensetzungen von 5 bis 30 Gew.-% beträgt, und dass der Anteil Fettsäure bezogen auf die Summe aus Fettsäure und Tensiden von 0,1 bis 20 Gew.-% beträgt, und dass die Zusammensetzung mindestens ein Verdickungsmittel enthält, wobei mindestens 90 Gew.-% der Verdickungsmittel ausgewählt sind aus Xanthan-Gum, Guar, Agar-Agar, Alginate, Tylosen, Cellulose, welche teilweise oder vollständig auf nachwachsenden Rohstoffen basieren.

Außerdem ist Gegenstand der vorliegenden Erfindung die Verwendung der erfindungsgemäßen Zusammensetzungen als oder zur Herstellung von Badezusätzen, Duschgel, Shampoos, Konditionierern, Imprägnierlösungen für feuchte Reinigungstücher, Körperreinigungsmitteln oder Hautreinigungsmitteln.

Ein Vorteil der erfindungsgemäßen Zusammensetzung besteht darin, dass der Anteil an in ihr vorhandenen Tensiden, die auf nachwachsenden Rohstoffen basieren, vorzugsweise mehr als 50 Gew.-% bezogen auf die Gesamtmenge der Tenside beträgt.

Ein weiterer Vorteil der erfindungsgemäßen Zusammensetzung besteht darin, dass als Rohstoffe für die Biotenside Zucker oder Zucker und Glyceride und/oder Fettsäuren verwendet werden können.

Ein weiterer Vorteil der erfindungsgemäßen Zusammensetzung ist, dass diese sehr mild ist.

Noch ein Vorteil ist, dass viele der erfindungsgemäßen Zusammensetzungen eine überraschend gute Performance in einem Paneltest (z.B. Emollienteigenschaften auf der Haut) erreichen im Vergleich zu Formulierungen mit Tensiden, die kein Biotensid sind und dabei häufig einen zu vernachlässigenden Effekt auf Viskosität und nahezu keinen Effekt auf das Schaumvermögen der untersuchten Tensidmischungen aufweisen.

Die erfindungsgemäßen Zusammensetzungen sowie deren Verwendungen werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere bezüglich der in Bezug genommenen Sachverhalte vollständig zum Offenbarungsgehalt der vorliegenden Erfindung gehören. Wenn im nachfolgenden Mittelwerte angegeben sind, so handelt es sich, wenn nicht anders angegeben, um zahlengemittelte Mittelwerte. Bei Prozentangaben handelt es sich, wenn nicht anders angegeben um Angaben in Gewichtsprozent. Werden nachfolgend Messwerte angegeben so wurden diese, wenn nicht anders angegeben, bei einer Temperatur von 25 °C und einem Druck von 1013 mbar bestimmt.

Die erfindungsgemäßen Zusammensetzungen enthaltend Wasser, mindestens ein Biotensid und mindestens eine Fettsäure, zeichnen sich dadurch aus, dass der Anteil der Summe aller Tenside in den erfindungsgemäßen Zusammensetzungen von 5 bis 30 Gew.-%, vorzugsweise 5 bis 25 Gew.-%, bevorzugt 5 bis 20 Gew.-% und besonders bevorzugt 10 bis 20 Gew.-% beträgt, und dass der Anteil an Fettsäuren bezogen auf die Summe aus Fettsäuren und Tensiden von 0,1 bis 20 Gew.-%, vorzugsweise 0,1 bis 15 und bevorzugt von 1 bis 10 Gew.-% beträgt.

Der Anteil an Biotensiden an der erfindungsgemäßen Zusammensetzungen beträgt vorzugsweise von 0,1 bis 30 Gew.-%, bevorzugt von 0,5 bis 20 Gew.-% und besonders bevorzugt von 0,75 bis 10 Gew.-% bezogen auf die Gesamtzusammensetzung.

Im Rahmen der vorliegenden Erfindung werden unter Biotensiden alle durch Fermentation hergestellten Glycolipide verstanden.

Als Rohstoffe für die Herstellung der Biotenside können Kohlenhydrate, insbesondere Zucker wie z.B. Glucose und/oder lipophile Kohlenstoff-Quellen wie Fette, Öle, Partialglyceride, Fettsäuren, Fettalkohole, langkettige gesättigte oder ungesättigte Kohlenwasserstoffe eingesetzt werden. Vorzugsweise sind in den erfindungsgemäßen Zusammensetzungen keine Biotenside vorhanden, die nicht durch Fermentation von Glycolipiden hergestellt werden, wie z. B. Lipoproteine

Bevorzugt weist die erfindungsgemäße Zusammensetzung als Biotenside Mono-, Di- oder Polyrhamnolipide und/oder Sophorolipide auf. Besonders bevorzugt weist die erfindungsgemäße Zusammensetzung eines oder mehrere der in EP 1 445 302 A mit den Formeln (I), (II) bzw. (III) beschriebenen Rhamnolipide bzw. Sophorolipide auf.

Besonders bevorzugt sind in der erfindungsgemäßen Zusammensetzung Fettsäuren vorhanden, die auf nachwachsenden Rohstoffen basieren, ausgewählt aus Ölsäure, Palmitinsäure, Stearinsäure und Linolsäure. Der Anteil an Fettsäuren, die nicht auf nachwachsenden Rohstoffen basieren, beträgt bezogen auf die Gesamtsumme der vorhandenen Fettsäuren vorzugsweise kleiner 10 Gew.-%, bevorzugt kleiner 1 Gew.-% und besonders bevorzugt kleiner 0,1 Gew.-%.

Die in der erfindungsgemäßen Zusammensetzung vorhandenen Fettsäuren können insbesondere solche sein, wie sie im Prozess der Herstellung des Biotensids ggf. eingesetzt werden bzw. ggf. als Nebenprodukt anfallen. Im Fall des Einsatzes eines Reaktionsgemisches, welches bei der Herstellung des Biotensids anfällt und welches Fettsäure aufweist, kann auf eine zusätzliche Zugabe von Fettsäure verzichtet werden, wenn der Anteil der Fettsäure den oben genannten Grenzen entspricht. Zudem kann, wenn Fettsäure im Reaktionsgemisch bei der Herstellung des Biotensids in geeigneten Mengen (wie beansprucht) anfällt, auf eine aufwändige Abtrennung dieser Fettsäure verzichtet werden.

Es kann vorteilhaft sein, wenn die erfindungsgemäße Zusammensetzung außerdem mindestens ein Tensid aufweist, welches kein Biotensid ist. Das Gewichtsverhältnis von Biotensiden zu Tensiden, die kein Biotensid sind kann in der erfindungsgemäßen Zusammensetzung >1 zu 1 oder <=1 zu 1 betragen. Vorzugsweise beträgt das Gewichtsverhältnis von Biotensiden zu Tensiden, die kein Biotensid >1 zu 1 oder <1 zu 1, bevorzugt >1 zu 1.

Als Tenside, die keine Biotenside sind, kann die erfindungsgemäße Zusammensetzung alle bekannten, insbesondere für kosmetische Formulierungen geeigneten Tenside aufweisen. Vorzugsweise werden solche Tenside eingesetzt, wie sie z. B. in DE 102005011785, EP 2000124 und WO 2010/108756 beschrieben werden.

Bevorzugt weist die erfindungsgemäße Zusammensetzung als Tenside, die keine Biotenside sind, Tenside mit anionischer, amphoterer/ampholytischer und/oder zwitterionischer Struktur auf. Typische Beispiele für milde, d. h. besonders hautverträgliche Tenside sind Fettalkoholpolyglycolethersulfate, Monoglyceridsulfate, Alkylsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Alkylpolyglucoside, Fettsäureglucamide, Alkylamidobetaine und/oder Alkylbetaine.

Als amphotere Tenside können z.B. Betaine, Amphoacetate oder Amphopropionate enthalten sein, so z.B. Substanzen wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat.

Als ampholytische Tenside können solche oberflächenaktiven Verbindungen vorhanden sein, die außer einer C_{8/18}-Alkyl- oder -Acylgruppe im Molekül mindestens eine freie Aminogruppe und mindestens eine -COOH- oder -SO₃H- Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Weitere Beispiele ampholytischer Tenside sind das N-Kokosalkylaminopropionat, das Kokosacylaminoethylaminopropionat und das C_{12/18}-Acylsarcosin.

Bevorzugte anionische Tenside sind z. B. die Salze verschiedener Kationen (Natrium, Ammonium oder andere) von Cocoylglutamate, Lauryl Glucose Carboxylate etc. Als zwitterionische Tenside können z. B. Cocamidopropylbetain oder Cocoamidopropylsultain in der Formulierung enthalten sein. Als amphotere Tenside sind insbesondere Amphoacetate wie Natriumcocoamphoacetat oder Dinatriumcocoamphodiacetat bevorzugt.

Bevorzugt sind Zusammensetzungen, die mindestens ein Tensid aus der Gruppe umfassend Laurylethersulfate, Alkylsulfate, Alkyloligoglucoside, Mono- und/oder Dialkylsulfosuccinate, Alkylamidobetaine oder Fettsäuresarcosinate als Nicht-Biotensid enthalten.

Besonders bevorzugte Zusammensetzungen enthalten keine Tenside, die Sulfate oder Polyethylenglykol aufweisen.

Die erfindungsgemäßen Zusammensetzungen können Farbstoffe aufweisen. Als Farbstoffe können z.B. die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S. 81 bis 106 zusammengestellt sind.

Es kann vorteilhaft sein, wenn die erfindungsgemäßen Zusammensetzungen einen oder mehrere Naturfarbstoffe aufweisen. Unter Naturfarbstoffen werden im Rahmen der vorliegenden Erfindung mineralische oder aus Pflanzen oder Tieren gewonnene Farbstoffe verstanden. In den erfindungsgemäßen Zusammensetzungen können alle Naturfarbstoffe eingesetzt werden. Bevorzugte natürlich vorkommende Farbstoffe sind z. B. Indigo, Lawson, Purpur, Karmin, Kermes, Alizarin, Waid, Crocetin, Brasilin, Safran, Crocetin, Kurkumia, Curcumin, Orlean, Bixin, Annato, Anthocyane, Betanin, Capsanthin, Carotin, Chlorophylle, Karminsäure, Luthein, Xanthophyll, Lycopin, Pflanzenkohle oder Zuckerkulör. Besonders bevorzugt werden Naturfarbstoffe eingesetzt, die Pflanzen oder Tieren gewonnen werden.

Besonders bevorzugt eingesetzte Naturstoffe sind Bixin (E 160b), Anthocyane (E 163), Betanin (E 162), Capsanthin (E 160c), Carotin (E 160a), Chlorophylle (E 140), Curcumin (E 100), Karminsäure (E 120), Luthein (E 161b), Xanthophyll, Lycopin (E 160d), Pflanzenkohle (E 153) und/oder Zuckerkulör (E 150a).

Besonders bevorzugt weist die erfindungsgemäße Zusammensetzung als Farbstoffe ausschließlich Naturfarbstoffe auf.

Der Anteil an Farbstoffen, bevorzugt Naturfarbstoffen an den erfindungsgemäßen Zusammensetzungen beträgt vorzugsweise von 0,001 bis 0,1 Gew.-%.

Durch die Verwendung von Naturfarbstoffen wird eine bessere biologische Abbaubarkeit und Verträglichkeit (Milde) der erfindungsgemäßen Zusammensetzung erreicht.

Die erfindungsgemäße Zusammensetzung kann Konservierungsstoffe aufweisen, z. B. solche, wie sie in der EG-Verordnung gelistet sind (Verordnung (EG) Nr. 1223/2009 des Europäischen Parlaments und des Rates vom 30. November 2009 über kosmetische Mittel, Annex V). Bevorzugte erfindungsgemäße Zusammensetzungen sind solche, die als Konservierungsmittel einen oder eine Kombination der folgenden Stoffe enthalten: Benzylalkohol, Natriumbenzoat, Kaliumsorbat, DMDM-Hydantoin, Ameisensäure, Benzoesäure oder Polyaminopropylbiguanid. Besonders bevorzugte Zusammensetzungen sind aber solche, die frei von Konservierungsmitteln, insbesondere frei von solchen gemäß der EG-Verordnung sind.

Es kann vorteilhaft sein, wenn die erfindungsgemäßen Zusammensetzungen Propylenglykol, Harnstoff, Glycerin, ätherische Öle, Phenylethylalkohol und/oder Ethanol enthält. Vorzugsweise beträgt der Anteil der Summe von Propylenglykol, Harnstoff, Glycerin, ätherische Öle, Phenylethylalkohol und Ethanol an der Zusammensetzung von 0,01 bis 15 Gew.-%, bevorzugt von 0,1 bis 10 Gew.-% beträgt. Durch die Verwendung einer oder mehrer der genannten Substanzen kann eine Vermehrung der Keimzahl verhindert oder zumindest vermindert werden.

Der Anteil an Verdickungsmitteln an der Zusammensetzung beträgt vorzugsweise von 0,05 bis 20 Gew.-%, bevorzugt von 0,1 bis 10 Gew.-% und besonders bevorzugt von 0,5 bis 5 Gew.-% bezogen auf die Zusammensetzung.

Erfindungsgemäße Zusammensetzungen sind solche, in denen ausschließlich Verdickungsmittel (also mindestens 90 Gew.-%, bevorzugt 100 Gew.-% der vorhandenen Verdickungsmittel) vorhanden sind, die teilweise oder vollständig, insbesondere vollständig, auf nachwachsenden Rohstoffen ausgewählt aus Xanthan-Gum, Guar, Agar-Agar, Alginate, Tylosen und Cellulose basieren.

Es kann vorteilhaft sein, wenn die erfindungsgemäße Zusammensetzung Parfümöle oder Duftstoffe aufweist. Als Parfümöle können alle bekannten und insbesondere die für die Herstellung von kosmetischen Formulierungen geeigneten Parfümöle eingesetzt werden. Geeignete Parfümöle können z. B. den Produktkatalogen bekannter Hersteller, wie beispielsweise Symrise, Frey&Lau oder IFF entnommen werden. Vorzugsweise weist die erfindungsgemäße Zusammensetzung Parfümöle oder Duftstoffe auf, wobei der Anteil der natürlichen Riechstoffe bezogen auf die Gesamtzahl der Riechstoffe in den Parfümölen mindestens 50 Gew.-%, vorzugsweise mindestens 75 % und bevorzugt mindestens 95 Gew.-% beträgt.

Als natürliche Riechstoffe können z. B. Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe als natürliche Riechstoffe in Frage, wie beispielsweise Zibet und Castoreum. Auch ätherische Öle eignen sich als Parfümöle, wie beispielsweise Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl, Lavandinöl, Bergamotteöl, Citronenöl, Mandarinenöl, Orangenöl, Muskateller Salbeiöl oder Geraniumöl.

Die erfindungsgemäßen Zusammensetzungen können als weitere Komponenten insbesondere z.B. solche enthalten, die z.B. ausgewählt aus der Gruppe der Emollients, Emulgatoren, Viskositätsregler/Stabilisatoren, UV-Lichtschutzfilter, Antioxidantien, Fest- und Füllstoffe, Filmbildner, Perlglanzadditive, Deodorant- und Antitranspirantwirkstoffe, Insektenrepellentien, Selbstbräuner, Konditioniermittel, (kosmetische) Wirkstoffe, Pflegeadditive, Überfettungsmittel, Lösungsvermittler (Solubilisatoren), Perlglanzmittel und Lösungsmittel sind, mit der Maßgabe, dass die genannten Komponenten nicht mit den Biotensiden, Tensiden, Verdickungsmitteln identisch sind. Substanzen, die als beispielhafte Vertreter der einzelnen Gruppen eingesetzt werden können, sind dem Fachmann bekannt und können beispielsweise den Anmeldungen DE 102008001788 A1, DE 102005011785 A1 und EP 2000124 A entnommen werden. Die dort als bevorzugt genannten Substanzen sollen auch im Rahmen der vorliegenden Erfindung als bevorzugte Substanzen gelten. Die Patentanmeldungen werden hiermit als Referenz eingeführt und gelten somit als Teil der Offenbarung. Bevorzugte erfindungsgemäße Zusammensetzungen weisen insbesondere Lösungsvermittler (Solubilisatoren) für wasserunlösliche Substanzen und Wirkstoffe zur Pflege von Haut und Haaren, wie z.B. Rückfetter auf. Der Anteil der Summe der in diesem Abschnitt genannten weiteren Komponenten an der erfindungsgemäßen Zusammensetzung beträgt vorzugsweise von 3 bis 50 Gew.-%, bevorzugt von 5 bis 25 Gew.-%.

Als Solubilisator oder auch Lösungsvermittler wird im Sinne der vorliegenden Erfindung eine Substanz bezeichnet, die in der Lage ist, wasserunlösliche Verbindungen möglichst klar in wässrigen Systemen in Lösung zu bringen. Nach allgemein akzeptierter Vorstellung werden bei diesem Vorgang Aggregate wie Mizellen gebildet, in deren Strukturen die hydrophoben Substanzen integriert sind. Optimal ist die Bildung einer "Mikroemulsion", also einer thermodynamisch stabilen Mischung aus Wasser (wässrige Lösung), einem Öl (nicht mit Wasser mischbare Substanz) und einem Solubilisator bzw. Lösungsvermittler. Typische Solubilisatoren sind ethoxylierte Fettderivate und Polyglycerin-Fettsäureester.

Als Pflegewirkstoffe oder Emollients können insbesondere z. B. Isopropylmyristat, Sucrose Cocoate, ethoxylierte Glycerin-Fettsäureester, wie beispielweise PEG-7 Glyceryl Cocoate (wie z.B. TEGOSOFT® GC erhältlich bei Evonik Industries AG), Polyglycerin-Fettsäureester wie Polyglyceryl-3 Caprate oder kationische Polymere, wie beispielsweise Polyquaternium-7 verwendet werden. Diese werden auch als Rückfetter bezeichnet.

Besonders bevorzugte erfindungsgemäße Zusammensetzungen sind frei von Polyethylenglykol (PEG) und/oder deren Derivate und vorzugsweise auch frei von Propylenglykol (PPG) und/oder deren Derivate.

Weiterhin besonders bevorzugte erfindungsgemäße Zusammensetzungen sind frei von Sulfaten und zudem frei von Polyethylenglykol (PEG) und/oder deren Derivate und Propylenglykol (PPG) und/oder deren Derivate.

Die erfindungsgemäßen Zusammensetzungen weisen vorzugsweise einen pH-Wert, bestimmt mittels eines pH-Meters vom Typ PB-11 der Firma Satorius von 5 bis 8 auf.

Bevorzugte Zusammensetzungen weisen eine Viskosität, bestimmt bei 25 °C mit einem Brookfield Viskometer Typ DV-I Prime von 500 bis 20.000 mPas auf.

Die erfindungsgemäßen Zusammensetzungen sind vorzugsweise ein Körper-, Haut- oder Haarbehandlungsmittel, zur Behandlung von Menschen und/oder Tieren, insbesondere Menschen und Säugetieren.

Eine erfindungsgemäße Zusammensetzung kann insbesondere ein Shampoo, ein Konditionierer, ein Duschgel, ein Körperreinigungsmittel oder ein Hautreinigungsmittel sein. Bevorzugt stellen erfindungsgemäße Zusammensetzungen flüssige, kosmetische, dermatologische oder pharmazeutische Körperreinigungsmittel, insbesondere Duschbäder und -gele, Badeformulierungen, Flüssigseifen und Shampoos dar oder werden für die Herstellung dieser Produkte/Formulierungen verwendet.

Die erfindungsgemäßen Zusammensetzungen können insbesondere ein Badzusatz, Shampoo, Konditionierer, Duschgel, Körperreinigungsmittel, Imprägnierlösungen für feuchte Reinigungstücher oder Hautreinigungsmittel sein oder zur Herstellung eines oder mehrerer dieser Produkte verwendet werden.

Besonders bevorzugte Zusammensetzungen sind solche, die mehrere oder alle der vorgenannten bevorzugten Merkmale in sich vereinen.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

Alle Konzentrationen in den Anwendungsbeispielen sind, wenn nicht anders angegeben, in Gewichtsprozent angegeben. Zur Herstellung der Zusammensetzungen wurden dem Fachmann bekannte, übliche Formulierungsverfahren eingesetzt.

### Produktbeispiel 1a: Sophorolipid:

Die Herstellung des Sophorolipids erfolgte mittels Fermentation mit der Hefe *Candida bombicola* auf Basis der Substrate Glucose, Sonnen-, Raps- oder Olivenöl (enthaltend als Fettsäureanteil hauptsächlich Ölsäure).

Das Wachstumsmedium enthielt folgende Bestandteile:
- 10,0 g/L Glucose ((D)+-Glucose · 1 H₂0)
- 7,5 g/L YNB (Yeast Nitrogen Base)
- 2,0 g/L Hefeextrakt
1,1 L des Mediums wurden in einem Fermenter mit 2 L Arbeitsvolumen autoklaviert und mit einer sich in der exponentiellen Phase befindlichen Vorkultur aus dem gleichen Medium angeimpft. Die Temperatur wurde auf 30 °C eingestellt. Der pO2 wurde über die Rührerdrehzahl auf 30 % relativer Sättigung bei Begasung mit Luft gehalten, die Rührerdrehzahl war jedoch nie kleiner als 200 rpm. Während der Biomassebildungsphase fiel der pH Wert auf 3,5 und wurde durch Zugabe von NaOH auf diesem Wert gehalten. Nachdem die Biomassebildungsphase beendet war (Verbrauch der vorhandenen Glukose, gekennzeichnet durch den Anstieg des pO₂, bzw. Abfall des pCO₂), wurde die Produktbildungsphase durch Zugabe von 150 g des entsprechenden Öls, 200 mL einer 750 g/L Glucoselösung und 10 mL einer 150 g/L Hefeextraktlösung eingeleitet. Das Ende der Produktbildungsphase war durch den erneuten Anstieg des pO2 gekennzeichnet. Nach Beendigung der Fermentation wurde der Ansatz autoklaviert, dabei setzte sich die Rohproduktphase ab. Die Rohproduktphase wurde mit Wasser gewaschen. Anschließend wurde die Produktphase mit Ethylacetat extrahiert und dann das Lösungsmittel unter Vakuum entfernt. Die Analytik mittels HPLC-MS und NMR ergab, dass das Produkt neben dem Sophorolipid noch ca. 10 Gew.-% nicht-umgesetzte freie Fettsäure (v.a. Ölsäure) bezogen auf Sophorolipide enthielt. Die restliche Fettsäure wurde aus dem Produkt mittels mehrfacher Extraktion mit n-Hexan bei pH 3 entfernt, so dass die Restmenge an freier Fettsäure <0,1 Gew.-% (HPLC) betrug. Es wurde ein farbloser Feststoff erhalten.

### Produktbeispiel 1b: Sophorolipid + 10% Ölsäure:.

Produktbeispiel 1a wurde mit 10 Gew.-% technischer Ölsäure (Oleic Acid, Cremer Oleo GmbH und Co. KG, Deutschland) versetzt.

### Produktbeispiel 2a: Rhamnolipid:

Produkt wie bei Firma Rhamnolipids, Inc., USA, als 25%ige wässrige Lösung erhältlich. Per HPLC-Analytik wird sichergestellt, dass keine freien Fettsäuren (d.h. <0,1 Gew.-% bezogen auf die Gesamtzusammensetzung des Produkts) als Nebenbestandteil beinhaltet sind.

### Produktbeispiel 2b: Rhamnolipid + 10% Ölsäure:

Produktbeispiel 2a wurde mit 10 Gew.-% technischer Ölsäure bzgl. des Rhamnolipids versetzt.

### Hautverträglichkeit nach RBC-Test:

Die Produkte 1a, 1b, 2a und 2b zeigen im sog. RBC-Test (Red Blood Cell-Test, siehe W. J. W. Pape, U. Pfannenbecker, U. Hoppe, Mol. Toxicol. 1987, 1, 525.) eine sehr gute Hautverträglichkeit. Die L/D-Werte sind >100. Die Produkte sind demnach als nicht reizend in diesem Test einzustufen.

### Anwendungstechnische Austestung:

### Austestung der Hautpflegeleistung und Schaumeigenschaften mittels eines Handwaschtests

Zur Bewertung der Hautpflegeleistung und der Schaumeigenschaften von Produktbeispielen 1a, 1b, 2a und 2b in wässrigen, tensidischen Zusammensetzungen (Tensidformulierungen) wurden sensorische Handwaschtests im Vergleich zum Sekundärtensid Cocamidopropyl Betaine durchgeführt. Cocamidopropyl Betaine ist in der Industrie als universell einsetzbares, mildes und schaum-förderndes Sekundärtensid sehr weit verbreitet und gilt als hochwirksame Komponente in wässrigen, tensidischen Formulierungen.

Eine Gruppe bestehend aus 10 trainierten Prüfpersonen wusch sich bei diesem Handwaschtest definiert die Hände und bewertete Schaumeigenschaften und Hautgefühl anhand einer Notenskala von 1 (schlecht) bis 5 (sehr gut). Die eingesetzten Produkte wurden jeweils in einer standardisierten Tensidformulierung (Tabelle 1) getestet.

Als Kontrollformulierung 3 wurde eine Tensidformulierung ohne Zusatz eines Sekundärtensids verwendet.

**Tabelle 1: Testformulierungen für Handwaschtest gemäß (Angaben in Gew.-%).**

| **Formulierungs-Beispiele** | **3** | **4** | **5** | **6** | **7** | **8** | **9** |
|---|---|---|---|---|---|---|---|
| Texapon® NSO (BASF Cognis, INCI: Sodium Laureth Sulfate, 28 %-ig) | 32,1 | 32,1 | 32,1 | 32,1 | 32,1 | 32,1 | 32,1 |
| TEGO® Betain F 50 (Evonik Industries AG, INCI: Cocamidopropyl Betaine, 38 %-ig) | | 7,9 | | | | | |
| **Produktbeispiel 1a:** | | | 3,0 | | | | |
| Sophorolipid (100%ig) | | | | | | | |
| **Produktbeispiel 1b:** | | | | 3,0 | | | 1,5 |
| 90% Sophorolipid + 10% Ölsäure | | | | | | | |
| **Produktbeispiel 2a:** Rhamnolipid (25%-ige wässrige Lösung) | | | | | 12,0 | | |
| **Produktbeispiel 2b:** | | | | | | 12,0 | 6,0 |
| 90% Rhamnolipid + 10% Ölsäure (25%ige wässrige Lösung) | | | | | | | |
| NaCl | 4,5 | 4,5 | 4,5 | 4,5 | 4,5 | 4,5 | 4,5 |
| Citric Acid, 30% | ad. pH 6,0 | | | | | | |
| Wasser, demineralisiert | ad. 100% | | | | | | |

In Tabelle 2 sind die Ergebnisse des Handwaschtests dargestellt.

**Tabelle 2: Ergebnisse des Handwaschtests**

| **Testformulierung** | **3** | **4** | **5** | **6** | **7** | **8** | **9** |
|---|---|---|---|---|---|---|---|
| Anschäumverhalten | 2,2 | 3,1 | 3,0 | 3,0 | 3,4 | 3,3 | 3,3 |
| Schaumcremigkeit | 2,0 | 2,8 | 2,4 | 2,3 | 3,0 | 3,0 | 2,9 |
| Hautgefühl während des Waschens | 2,9 | 3,3 | 3,4 | 3,6 | 3,4 | 3,5 | 3,5 |
| Leichtigkeit des Abwaschens | 4,0 | 4,1 | 4,4 | 4,5 | 4,0 | 4,2 | 4,4 |
| Hautglätte nach 3 min. | 2,8 | 3,1 | 3,0 | 3,2 | 3,3 | 3,6 | 3,5 |
| Hautweichheit nach 3 min. | 3,0 | 3,1 | 3,0 | 3,2 | 3,4 | 3,5 | 3,4 |

Anhand der Messergebnisse in Tabelle 2 wird ersichtlich, dass die Zusammensetzung 6 unter Verwendung von Produktbeispiel 1b (Sophorolipid + 10% Ölsäure) ein besseres Hautgefühl während des Waschens mit der Formulierung und überraschenderweise eine leichtere Abwaschbarkeit im Vergleich zu der Vergleichszusammensetzung 4 nach dem Stand der Technik aufweist. Die leichte Abwaschbarkeit (Rinseability) ist ein immer wichtig werdender Parameter bei der Entwicklung neuer umweltfreundlicher Kosmetikprodukte, da Wassereinsparungen während des Waschens zu wesentlich besseren Ökobilanzen führen. Zudem wird ersichtlich, dass die Zusammensetzung 6 bei Hautgefühl, Hautglätte und Hautweichheit besser bewertet wurde als Testformulierung 5 ohne Ölsäure. Überraschenderweise werden dabei die Schaumeigenschaften aber kaum beeinflusst.

Die Kontrollformulierung 3 weist in allen Eigenschaften die schlechtesten Werte auf.

Des Weiteren ist anhand der Messwerte ersichtlich, dass die Zusammensetzung 8 mit Produktbeispiel 2b (Rhamnolipid + 10% Ölsäure) und auch die Formulierung 9 mit beiden Produktbeispielen (1b und 2b, 50:50) überraschenderweise eine deutliche Verbesserung der Schaumeigenschaften und des Hautgefühls während des Waschens und nach der Applikation und Trocknen im Vergleich zum Stand der Technik (Formulierung 4) bewirkt. Im Vergleich zu Formulierung 7 (Rhamnolipid ohne Ölsäure) zeigten Formulierungen 8 und 9 speziell bei Hautglätte und bei der Abwaschbarkeit Vorteile.

Wider Erwarten zeigte in den vorliegenden Formulierungen ein gewisser Anteil an freier Fettsäure im Biotensid keinen negativen Einfluss auf das Schaumverhalten oder die Verdickungseigenschaften.

### Weitere Formulierungsbeispiele:

Die in den nachfolgenden Tabellen angegebenen Formulierungsbeispiele zeigen exemplarische Vertreter einer Vielzahl von möglichen Zusammensetzungen, wobei nur solche erfindungsgemäß sind, die auch alle Merkmale des vorliegenden Anspruchs 1 vorweisen.

Die einsetzten Rohstoffe sind mit der INCI-Bezeichnung aufgeführt.

Wenn nicht anders angegeben, handelt es sich bei den Angaben in den Tabellen um Angaben in Gew.-%. Die Einsatzkonzentrationen sind in Gew.-% an Aktivstoff angegeben.

Falls die Herstellung der Formulierung zuvor die getrennte Zubereitung bzw. Mischung von Formulierungsbestandteilen erfordert, wird dieses als mehrphasige Zubereitung bezeichnet. Falls eine zweiphasige Herstellung erforderlich ist, werden die beiden Phasen mit A und B in den angegebenen Tabellen gekennzeichnet. Bei drei- oder vierphasigen Prozessen werden die Phasen mit A, B, C bzw. D benannt.

### Laurylethersulfat-basierte Systeme:

**Tabelle 3: Conditioning Anti-Schuppen Shampoo**

| | | |
|---|---|---|
| A | Glycol Distearate | 3,0% |
| | Sodium Laureth Sulfate | 8,0% |
| B | Parfum | q.s. |
| | Zinc Pyrithione | 1,0% |
| | Silicone Quaternium-22 | 1,0% |
| C | Water | ad 100% |
| | Acrylates/ C10-30 Alkyl Acrylate Crosspolymer | 0,2% |
| | Polyquaternium-10 | 0,3% |
| | NaOH, 25% | 0,3% |
| D | Undecylenamidopropyl Betaine | 2,5% |
| | Produktbeispiel 1b: Sophorolipid + 10% Ölsäure | 2,5% |
| | Preservative | q.s |

**Tabelle 4: Conditioning Anti-Schuppen Shampoo**

| | | |
|---|---|---|
| A | Glycol Distearate | 3,0% |
| | Sodium Laureth Sulfate | 10,0% |
| B | Parfum | q.s. |
| | Zinc Pyrithione | 1,0% |
| | Quaternium-80 | 0,5% |
| C | Water | ad 100% |
| | Carbomer | 0,5% |
| | Polyquaternium-10 | 0,3% |
| | NaOH, 25% | 0,8% |
| D | Undecylenamidopropyl Betaine | 3,0% |
| | Produktbeispiel 2b: Rhamnolipid + 10% Ölsäure | 2,0% |
| | Preservative | q.s. |

**Tabelle 5: Conditioning Anti-Schuppen Shampoo**

| | | |
|---|---|---|
| A | Glycol Distearate | 3,0% |
| | Sodium Laureth Sulfate | 9,0% |
| B | Parfum | q.s |
| | Climbazol | 0,1% |
| | Quaternium-80 | 1,0% |
| C | Water | ad 100% |
| | Acrylates/ C10-30 Alkyl Acrylate Crosspolymer | 0,3% |
| | Polyquaternium-10 | 0,2% |
| | NaOH, 25%ig | 0,3% |
| D | Undecylenamidopropyl Betaine | 3,5% |
| | Produktbeispiel 1b: Sophorolipid + 10% Ölsäure | 1,5% |
| | Preservative | q.s |

**Tabelle 6: Conditioning Anti-Schuppen Shampoo**

| | | |
|---|---|---|
| A | PEG-3 Distearate | 2,5% |
| | Sodium Laureth Sulfate | 10,0% |
| B | Parfum | q.s. |
| | Octopirox | 0,3% |
| | Dimethicone | 0,5% |
| C | Water | ad 100% |
| | Acrylates/ C10-30 Alkyl Acrylate Crosspolymer | 0,2% |
| | Polyquaternium-10 | 0,3% |
| | NaOH, 25% | 0,3% |
| D | Undecylenamidopropyl Betaine | 2,0% |
| | Produktbeispiel 2b: Rhamnolipid + 10% Ölsäure | 3,0% |
| | Preservative, Dyes | q.s |

**Tabelle 7: Feuchtigkeitspendendes Hautreinigungsmittel**

| | | |
|---|---|---|
| A | Sodium Laureth Sulfate | 8,0% |
| | Produktbeispiel 1b: Sophorolipid + 10% Ölsäure | 1,0% |
| | Parfum | q.s. |
| B | Water | ad 100% |
| | Hydroxypropyl Methylcellulose | 1,2% |
| | Cocamidopropyl Betaine | 3,0% |
| | PPG-3 Myristyl Ether | 1,0% |
| | Glycol Distearate | 2,0% |
| | PEG-200 Hydrogenated Glyceryl Palmate (and) PEG-7 Glyceryl Cocoate | 1,0% |
| | Preservative | q.s. |
| | Citric Acid, 30% | ad pH 5,5 |

**Tabelle 8: Feuchtigkeitspendendes Hautreinigungsmittel**

| | | |
|---|---|---|
| A | Sodium Laureth Sulfate | 7,0% |
| | Produktbeispiel 2b: Rhamnolipid + 10% Ölsäure | 3,0% |
| | Parfum | q.s. |
| B | Water | ad 100% |
| | Hydroxypropyl Methylcellulose | 1,2% |
| | Cocamidopropyl Betaine | 2,0% |
| | PPG-3 Myristyl Ether | 1,0% |
| | PEG-3 Distearate | 1,0% |
| | PEG-200 Hydrogenated Glyceryl Palmate (and) PEG-7 Glyceryl Cocoate | 1,0% |
| | Preservative | q.s. |
| | Citric Acid, 30% | ad pH 5,7 |

**Tabelle 9: Körperreinigungsmittel**

| | | |
|---|---|---|
| A | Sodium Laureth Sulfate | 8,5% |
| | Produktbeispiel 2b: Rhamnolipid + 10% Ölsäure | 1,5% |
| | Bis-PEG/PPG-20/20 Dimethicone | 0,3% |
| | Parfum | q.s. |
| B | Water | ad 100% |
| | Hydroxypropyl Methylcellulose | 1,2% |
| | Sodium Cocoamphoacetate | 2,5% |
| | PEG-3 Distearate | 2,0% |
| | PEG-200 Hydrogenated Glyceryl Palmate (and) PEG-7 Glyceryl Cocoate | 1,6% |
| | Preservative | q.s. |
| | Citric Acid, 30% | ad pH 5,8 |

**Tabelle 10: Körperreinigungsmittel**

| | | |
|---|---|---|
| A | Sodium Laureth Sulfate | 8,0% |
| | Produktbeispiel 1b: Sophorolipid + 10% Ölsäure | 1,2% |
| | Bis-PEG/PPG-20/20 Dimethicone | 0,3% |
| | Parfum | q.s. |
| B | Water | ad 100% |
| | Hydroxypropyl Methylcellulose | 1,2% |
| | Citric Acid Monohydrate | 0,5% |
| | Sodium Cocoamphoacetate | 3,0% |
| | Glycol Distearate (and) Laureth-4 (and) Cocamidopropyl Betaine | 2,0% |
| | PEG-200 Hydrogenated Glyceryl Palmate (and) PEG-7 Glyceryl Cocoate | 1,6% |
| | Preservative | q.s. |
| | Citric Acid, 30% | ad pH 5,5 |

**Tabelle 11: Körperreinigungsschaum**

| | |
|---|---|
| Sodium Laureth Sulfate | 3,5% |
| Produktbeispiel 2b: Rhamnolipid + 10% Ölsäure | 3,0% |
| Sodium Cocoamphoacetate | 3,0% |
| Water | ad 100% |
| Hydroxypropyl Methylcellulose | 0,5% |
| Sodium Lactate (and) Sodium PCA (and) Glycine (and) Fructose (and) Urea (and) Niacinamide (and) Inositol (and) Sodium Benzoate (and) Lactic Acid | 1,0% |
| Citric Acid | ad pH 5,5 |
| Parfum | q.s. |

**Tabelle 12: Körperreinigungsschaum**

| | |
|---|---|
| Sodium Laureth Sulfate | 3,5% |
| Produktbeispiel 1b: Sophorolipid + 10% Ölsäure | 3,6% |
| Produktbeispiel 2b: Rhamnolipid + 10% Ölsäure | 0,7% |
| Sodium Cocoamphoacetate | 2,0% |
| Water | ad 100% |
| Hydroxypropyl Methylcellulose | 0,5% |
| Sodium Lactate (and) Sodium PCA (and) Glycine (and) Fructose (and) Urea (and) Niacinamide (and) Inositol (and) Sodium Benzoate (and) Lactic Acid | 0,5% |
| Panthenol | 0,2% |
| Citric Acid Monohydrate | ad pH 4,9 |
| Parfum | q.s. |

**Tabelle 13: Klares Conditioning Shampoo**

| | |
|---|---|
| Sodium Laureth Sulfate | 9,0% |
| Palmitamidopropyltrimonium Chloride | 1,0% |
| PEG-200 Hydrogenated Glyceryl Palmate (and) PEG-7 Glyceryl Cocoate | 2,0% |
| Produktbeispiel 2b: Rhamnolipid + 10% Ölsäure | 0,7% |
| Water | ad 100% |
| Creatine | 1,0% |
| Hydroxypropyl Guar Hydroxypropyltrimonium Chloride | 0,2% |
| Cocamidopropyl Betaine | 2,0% |
| NaCl | 0,5% |
| Parfum | q.s. |
| Preservative | q.s. |

**Tabelle 14: Klares Conditioning Shampoo**

| | |
|---|---|
| Sodium Laureth Sulfate | 8,0% |
| PEG-200 Hydrogenated Glyceryl Palmate (and) PEG-7 Glyceryl Cocoate | 2,0% |
| Produktbeispiel 1b: Sophorolipid + 10% Ölsäure | 1,5% |
| Water | ad 100% |
| Polyquaternium-10 | 0,2% |
| Cocamidopropyl Betaine | 3,0% |
| NaCl | 0,3% |
| Parfum | q.s. |
| Preservative | q.s. |

**Tabelle 15: Trübes Conditioning Shampoo**

| | |
|---|---|
| Sodium Laureth Sulfate | 8,0% |
| PEG-200 Hydrogenated Glyceryl Palmate (and) PEG-7 Glyceryl Cocoate) | 2,0% |
| Produktbeispiel 1b: Sophorolipid + 10% Ölsäure | 1,5% |
| Produktbeispiel 2b: Rhamnolipid + 10% Ölsäure | 0,7% |
| Water | ad 100% |
| Polyquaternium-10 | 0,2% |
| Cocamidopropyl Betaine | 1,5% |
| Dimethicone (and) Dimethiconol | 1,0% |
| Glycol Distearate (and) Laureth-4 (and) Cocamidopropyl Betaine) | 2,0% |
| NaCl | 0,3% |
| Parfum | q.s. |
| Preservative | q.s. |

**Tabelle 16: Körperreinigungsmittel mit Perleffekt**

| | |
|---|---|
| Sodium Laureth Sulfate | 9,0% |
| Produktbeispiel 1b: Sophorolipid + 10% Ölsäure | 2,5% |
| Water | ad 100% |
| Cocamidopropyl Betaine | 1,5% |
| Glycol Distearate (and) Laureth-4 (and) Cocamidopropyl Betaine | 2,0% |
| PEG-18 Glyceryl Oleate/Cocoate | 1,5% |
| NaCl | 0,5% |
| Parfum | q.s. |
| Preservative | q.s. |

**Tabelle 17: Duschgel**

| | |
|---|---|
| Sodium Laureth Sulfate | 4,0% |
| Produktbeispiel 2b: Rhamnolipid + 10% Ölsäure | 2,5% |
| Sodium Cocoyl Hydrolyzed Wheat Protein Glutamate | 1,5% |
| Disodium PEG-5 Laurylcitrate Sulfosuccinate (and) Capryl/Capramidopropyl Betaine | 2,5% |
| Water | ad 100% |
| Cocamidopropyl Betaine | 3,0% |
| Capryl/Capramidopropyl Betaine | 1,0% |
| Polyquaternium-7 | 0,5% |
| PEG-200 Hydrogenated Glyceryl Palmate (and) PEG-7 Glyceryl Cocoate | 1,8% |
| Parfum | q.s. |
| Preservative | q.s. |

**Tabelle 18: Duschgel**

| | |
|---|---|
| Water | ad 100% |
| Sodium Laureth Sulfate | 5,0% |
| Produktbeispiel 1b: Sophorolipid + 10% Ölsäure | 2,5% |
| Sodium Cocoyl Hydrolyzed Wheat Protein Glutamate | 1,5% |
| Disodium PEG-5 Laurylcitrate Sulfosuccinate (and) Capryl/Capramidopropyl Betaine | 2,5% |
| Cocamidopropyl Betaine | 3,0% |
| Capryl/Capramidopropyl Betaine | 2,0% |
| PEG-200 Hydrogenated Glyceryl Palmate (and) PEG-7 Glyceryl Cocoate | 1,8% |
| Preservative, Parfum, Dyes | q.s. |

**Tabelle 19: Duschgel**

| | |
|---|---|
| Water | ad 100% |
| Sodium Laureth Sulfate | 9,0% |
| Produktbeispiel 1b: Sophorolipid + 10% Ölsäure | 4,0% |
| Sodium Cocoamphoacetate | 1,5% |
| Capryl/Capramidopropyl Betaine | 1,5% |
| PEG-200 Hydrogenated Glyceryl Palmate (and) PEG-7 Glyceryl Cocoate | 0,5% |
| Citric Acid, 30% | ad pH 6,1 |
| Preservative, Parfum | q.s. |

**Tabelle 20: Klares Duschgel**

| | |
|---|---|
| Water | ad 100% |
| Sodium Laureth Sulfate | 11,0% |
| Produktbeispiel 2b: Rhamnolipid + 10% Ölsäure | 2,0% |
| PEG-40 Hydrogenated Castor Oil | 2,5% |
| Cocamidopropyl Betaine | 2,0% |
| Sodium Lactate (and) Sodium PCA (and) Glycine (and) Fructose (and) Urea (and) Niacinamide (and) Inositol (and) Sodiumbenzoate (and) Lactic Acid | 1,0% |
| PEG-18 Glyceryl Oleate/Cocoate | 2,0% |
| Preservative, Parfum | q.s. |

**Tabelle 21: Haar- und Körperreinigungsmittel**

| | |
|---|---|
| Sodium Laureth Sulfate | 7,0% |
| Coco-Betaine | 3,0% |
| Produktbeispiel 1b: Sophorolipid + 10% Ölsäure | 1,5% |
| Produktbeispiel 2b: Rhamnolipid + 10% Ölsäure | 1,5% |
| Polyquaternium-10 | 0,2% |
| PEG-150 Distearate | 1,5% |
| Hydrogenated Didecene | 1,5% |
| Water | ad 100% |
| Citric Acid | ad pH 6,0 |
| Preservative, Parfum, Dyes | q.s. |

**Tabelle 22: Waschlotion für empfindliche Haut**

| | |
|---|---|
| Water | ad 100% |
| Sodium Laureth Sulfate | 7,5% |
| Cocamidopropyl Betaine | 3,0% |
| Glyceryl Oleate | 3,0% |
| Produktbeispiel 1b: Sophorolipid + 10% Ölsäure | 2,0% |
| Sodium Chloride | 2,0% |
| Glycol Distearate | 1,0% |
| Sodium Lactate | 1,0% |
| Disodium Cocoyl Glutamate | 1,0% |
| Starch Hydroxypropyltrimonium Chloride | 0,5% |
| Glyceryl Stearate | 0,4% |
| Allantoin | 0,1% |
| Isopropyl Alcohol | 0,1% |
| Alcohol | 0,1% |
| Sorbitol | 0,1% |
| Urea | 0,2% |
| Serine | 0,05% |
| Tocopherol | 0,05% |
| Citric Acid | ad pH 5,5 |
| Preservative, Parfum | q.s. |

**Tabelle 23: Anti-Schuppen Shampoo**

| | |
|---|---|
| Water | ad 100% |
| Sodium Laureth Sulfate | 7,0% |
| Sodium Lauryl Sulfate | 4,0% |
| Produktbeispiel 1b: Sophorolipid + 10% Ölsäure | 3,0% |
| Cocamide MEA | 2,5% |
| Zinc Carbonate | 1,0% |
| Glycol Distearate | 1,0% |
| Sodium Chloride | 1,0% |
| Zinc Pyrithione | 1,0% |
| Dimethicone | 1,0% |
| Cetyl Alcohol | 0,5% |
| Polyquaternium-10 | 0,2% |
| Sodium Xylenesulfonate | 0,2% |
| Ammonium Laureth Sulfate | 0,2% |
| Sodium Diethylenetriamine-Pentamethylene Phosphonate | 0,1% |
| Etidronic Acid | 0,1% |
| DMDM Hydantoin | 0,1% |
| Disodium EDTA | 0,1% |
| Tetrasodium EDTA | 0,05% |
| Preservative, Parfum, Dyes | q.s. |

**Tabelle 24: Shampoo**

| | |
|---|---|
| Water | ad 100% |
| Sodium Laureth Sulfate | 5,0% |
| Produktbeispiel 2b: Rhamnolipid + 10% Ölsäure | 4,0% |
| Cocamidopropyl Betaine | 2,0% |
| Sodium Chloride | 2,0% |
| PEG-3 Distearate | 1,5% |
| Polyquaternium-10 | 0,3% |
| Citrus Aurantifolia Fruit Juice | 0,2% |
| Paulinia Cupana Extract | 0,2% |
| PEG-40 Hydrogenated Castor Oil | 0,2% |
| Propylene Glycol | 0,2% |
| Glycerin | 0,2% |
| Citric Acid | ad pH 5,0 |
| Preservative, Parfum | q.s. |

**Tabelle 25: Shampoo**

| | |
|---|---|
| Water | ad 100% |
| Sodium Laureth Sulfate | 7,0% |
| Cocamidopropyl Betaine | 2,5% |
| Produktbeispiel 1b: Sophorolipid + 10% Ölsäure | 1,5% |
| Sodium Chloride | 1,5% |
| Glycol Distearate | 1,0% |
| Hydroxypropyl Guar Hydroxypropyltrimonium Chloride | 0,3% |
| Propylene Glycol | 0,3% |
| Panthenol | 0,3% |
| Dimethicone Propyl PG-Betaine | 0,3% |
| Glyoxylic Acid | 0,02% |
| Coumarin | 0,01% |
| Citric Acid | ad pH 5,5 |
| Preservative, Parfum | q.s. |

**Tabelle 26: Shampoo**

| | |
|---|---|
| Water | ad 100% |
| Sodium Laureth Sulfate | 5,0% |
| TEA-cocoyl Glutamate | 3,0% |
| Glycerin | 3,0% |
| Produktbeispiel 2b: Rhamnolipid + 10% Ölsäure | 2,0% |
| Sodium Chloride | 2,0% |
| Coco-Betaine | 1,0% |
| Cocamide MIPA | 0,5% |
| Glycol Distearate | 0,5% |
| Sodium Methyl Cocoyl Taurate | 0,4% |
| Sodium PCA | 0,2% |
| Hexylene Glycol | 0,1% |
| Polyquaternium-10 | 0,1% |
| Disodium Cocoamphodiacetate | 0,2% |
| Ceteareth-60 Myristyl Glycol | 0,2% |
| Brassica Campestris Seed Oil (Seed) | 0,1% |
| Citric Acid | ad pH 5,5 |
| Preservative, Parfum | q.s. |

**Tabelle 27: Shampoo**

| | |
|---|---|
| Water | ad 100% |
| Sodium Laureth Sulfate | 5,0% |
| Produktbeispiel 1b: Sophorolipid + 10% Ölsäure | 3,5% |
| Sodium Laureth-4 Carboxylate | 1,5% |
| Dipropylene Glycol | 1,5% |
| Sodium Cocoamphoacetate | 1,0% |
| Glycol Distearate | 0,8% |
| Lauramide MIPA | 0,5% |
| C12-14 Hydroxyalkyl Hydroxyethyl Sarcosine | 0,5% |
| Dimethicone | 0,4% |
| Polyquaternium-10 | 0,2% |
| Polyquaternium-7 | 0,1% |
| Sodium Citrate | 0,1% |
| Hydrolyzed Collagen | 0,1% |
| Laureth-25 | 0,1% |
| Rosa Canina Fruit Oil | 0,1% |
| Disodium EDTA | 0,1% |
| Citric Acid | ad pH 5,5 |
| Preservative, Parfum | q.s. |

**Tabelle 28: Duschgel**

| | |
|---|---|
| Water | ad 100% |
| Produktbeispiel 1b: Sophorolipid + 10% Ölsäure | 4,2% |
| Sodium Laureth Sulfate | 4,0% |
| Cocamidopropyl Betaine | 2,5% |
| Glycerin | 2,0% |
| Sodium Chloride | 1,5% |
| Sodium Myristoyl Sarcosinate | 1,2% |
| Prunus Cerasus Juice | 0,7% |
| Caramel | 0,2% |
| Disodium EDTA | 0,1% |
| Propylene Glycol | 0,2% |
| PEG-120 Methyl Glucose Trioleate | 0,3% |
| Citric Acid | ad pH 5,5 |
| Preservative, Parfum | q.s. |

**Tabelle 29: Duschgel**

| | |
|---|---|
| Water | ad 100% |
| Ammonium Laureth Sulfate | 5,5% |
| Cocamidopropyl Betaine | 2,5% |
| Produktbeispiel 1b: Sophorolipid + 10% Ölsäure | 1,5% |
| Sorbitol | 1,2% |
| Cocamide Methyl MEA | 0,7% |
| PEG-7 Glyceryl Cocoate | 0,6% |
| Sodium Cocoyl Alaninate | 0,8% |
| Sodium Chloride | 0,7% |
| DMDM Hydantoin | 0,1% |
| Disodium EDTA | 0,1% |
| Santalum Album Extract (Extract) | 0,1% |
| Lactic Acid, 90% | ad pH 5,5 |
| Preservative, Parfum | q.s. |

**Tabelle 30: Handreinigungspaste**

| | |
|---|---|
| Water | ad 100% |
| Laureth-5 | 5,0% |
| Juglans Regia | 3,0% |
| Sodium Laureth Sulfate | 5,0% |
| Produktbeispiel 1b: Sophorolipid + 10% Ölsäure | 2,0% |
| Bentonite | 1,0% |
| Sodium Cocoamphoacetate | 0,7% |
| Oleic Acid | 0,5% |
| C12-13 Alkyl Lactate | 0,5% |
| Aloe Barbadensis | 0,3% |
| Sodium Chloride | 0,3% |
| PEG-14M | 0,2% |
| Citric Acid | ad pH 6,0 |
| Preservative, Parfum | q.s. |

**Tabelle 31: Duschöl**

| | |
|---|---|
| Glycerin Soja Oil | 40,0% |
| Lecithin | 20,0% |
| MIPA-Laureth Sulfate | 4,0% |
| Produktbeispiel 1b: Sophorolipid + 10% Ölsäure | 4,0% |
| Laureth-4 | 3,0% |
| Cocamide DEA | 1,0% |
| Poloxamer 101 | 1,0% |
| Persea Gratissima Oil | 1,0% |
| Sodium Lactate | 0,5% |
| Water | ad 100% |
| Caprylic/Capric Triglyceride | 0,7% |
| Ascorbyl Palmitate | 0,5% |
| Sodium Citrate | 0,5% |
| Preservative, Parfum | q.s. |

### Alkylsulfat-basierte Systeme:

**Tabelle 32: Pflegedusche**

| | |
|---|---|
| Water | ad 100% |
| Ammonium Lauryl Sulfate | 5,0% |
| Lauryl Glucoside | 3,0% |
| Cocamidopropyl Betaine | 2,5% |
| Produktbeispiel 1b: Sophorolipid + 10% Ölsäure | 2,0% |
| Lauroyl Sarcosine | 0,7% |
| Bambusa Arundinacea Extract | 1,0% |
| Citrus Grandis Extract | 1,0% |
| Alcohol | 0,7% |
| Hydroxypropyl Guar Hydroxypropyltrimonium Chloride | 0,3% |
| Citrus Medica Limonum Oil | 0,3% |
| Preservative, Parfum | q.s. |

**Tabelle 33: Baby Waschlotion und Shampoo**

| | |
|---|---|
| Water | ad 100% |
| Sodium Coco Sulfate | 5,5% |
| Glycerin | 3,5% |
| Produktbeispiel 1b: Sophorolipid + 10% Ölsäure | 3,0% |
| Decyl Glucoside | 1,5% |
| Alcohol | 1,0% |
| Xanthan Gum | 1,0% |
| Bellis Perennis Extract | 0,7% |
| Arnica Montana Extract | 0,7% |
| Chamomilla Recutita Extract | 0,5% |
| Disodium Cocoyl Glutamate | 0,5% |
| Sodium Cocoyl Glutamate | 0,3% |
| Preservative, Parfum | q.s. |

**Tabelle 34: Shampoo**

| | |
|---|---|
| Water | ad 100% |
| Ammonium Lauryl Sulfate | 3,0% |
| Ammonium Laureth Sulfate | 2,5% |
| Cocamidopropyl Betaine | 2,0% |
| Produktbeispiel 2b: Rhamnolipid + 10% Ölsäure | 1,5% |
| Sodium Cocoyl Isethionate | 1,0% |
| Ammonium Chloride | 0,8% |
| Sodium Methyl Cocoyl Taurate | 0,7% |
| Sodium Lauroamphoacetate | 0,5% |
| Glycol Distearate | 0,5% |
| Laureth-7 | 0,4% |
| Propylene Glycol | 0,4% |
| Guar Hydroxypropyltrimonium Chloride | 0,2% |
| Dipropylene Glycol | 0,2% |
| Tetrasodium EDTA | 0,1% |
| Sodium Xylene Sulfonate | 0,1% |
| PEG-20 Castor Oil | 0,2% |
| Sodium Cocoamphoacetate | 0,2% |
| Quaternium-80 | 0,3% |
| Persea Gratissima Oil | 0,3% |
| Citric Acid, 30% | ad pH 5,5 |
| Preservative, Parfum | q.s. |

### Betain-basierte Systeme:

**Tabelle 35: Mildes, sulfat-freies Haar- und Körperreinigungsmittel**

| | |
|---|---|
| Cocamidopropyl Betaine | 5,0% |
| Sodium Cocoamphoacetate | 4,0% |
| Produktbeispiel 2b: Rhamnolipid + 10% Ölsäure | 1,5% |
| Sucrose Cocoate | 1,5% |
| PEG-120 Methyl Glucose Dioleate | 2,0% |
| Polyquaternium-10 | 0,2% |
| Water | ad 100% |
| Citric Acid, 30% | ad pH 6,0 |
| Preservative, Parfüm, Dyes | q.s. |

**Tabelle 36: Mildes, PEG-freies Haar- und Körperreinigungsmittel**

| | |
|---|---|
| Water | ad 100% |
| Cocamidopropyl Betaine | 5,5% |
| Lauryl Glucoside | 3,0% |
| Sodium Cocoamphoacetate | 3,0% |
| Produktbeispiel 1b: Sophorolipid + 10% Ölsäure | 1,5% |
| Hydroxypropyl Methylcellulose | 0,5% |
| Isostearamide MIPA (and) Glyceryl Laurate | 1,4% |
| Polyquaternium-7 | 0,5% |
| Citric Acid, 30% | ad pH 5,0 |
| Parfüm, Preservative | q.s. |

**Tabelle 37: Pflegedusche für empfindliche Haut**

| | |
|---|---|
| Water | ad 100% |
| Cocamidopropyl Betaine | 5,0% |
| Sodium Myreth Sulfate | 3,0% |
| Sodium Laureth Sulfate | 1,5% |
| Produktbeispiel 1b: Sophorolipid + 10% Ölsäure | 1,5% |
| PEG-7 Glyceryl Cocoate | 1,5% |
| Glycerin | 1,0% |
| Glyceryl Glucoside | 1,0% |
| Bisabolol | 0,3% |
| Chamamilla Recutica Flower Extract | 0,3% |
| Sodium Chloride | 0,3% |
| PEG-40 Hydrogenated Castor Oil | 0,3% |
| Polyquaternium-7 | 0,3% |
| PEG-90 Glyceryl Isostearate | 0,3% |
| PEG-200 Hydrogenated Glyceryl Palmate | 0,3% |
| Benzophenone-4 | 0,1% |
| Laureth-2 | 0,1% |
| Citric Acid | ad pH 4,9 |
| Preservative, Parfum | q.s. |

**Tabelle 38: Baby Shampoo**

| | |
|---|---|
| Water | ad 100% |
| Cocamidopropyl Betaine | 6,0% |
| PEG-80 Sorbitan Laurate | 3,0% |
| Produktbeispiel 2b: Rhamnolipid + 10% Ölsäure | 2,0% |
| Sodium Trideceth Sulfate | 1,5% |
| PEG-150 Distearate | 1,5% |
| Tetrasodium EDTA | 0,5% |
| Polyquaternium-10 | 0,2% |
| Quaternium-15 | 0,2% |
| Sodium Hydroxide | 0,3% |
| Citric Acid | ad pH 5,9 |
| Preservative, Parfüm, Dyes | q.s. |

**Tabelle 39: Baby Shampoo**

| | |
|---|---|
| Water | ad 100% |
| Cocamidopropyl Betaine | 7,0% |
| Produktbeispiel 1b: Sophorolipid + 10% Ölsäure | 2,5% |
| Sodium Laureth Sulfate | 1,5% |
| PEG-120 Methyl Glucose Dioleate | 1,0% |
| Coco Glucoside | 1,0% |
| Glyceryl Oleate | 0,7% |
| Quaternium-22 | 0,3% |
| C12-15 Alkyl Lactate | 0,2% |
| Quaternium-26 | 0,2% |
| Citric Acid | ad pH 5,5 |
| Preservative, Parfüm, Dyes | q.s. |

**Tabelle 40: Baby Shampoo**

| | |
|---|---|
| Water | ad 100% |
| PEG-80 Sorbitan Laurate | 3,5% |
| Cocamidopropyl Betaine | 3,5% |
| Sodium Trideceth Sulfate | 3,5% |
| Glycerin | 1,0% |
| Produktbeispiel 1b: Sophorolipid + 10% Ölsäure | 0,9% |
| Lauroamphoglycinate | 0,7% |
| PEG-150 Distearate | 0,5% |
| Sodium Laureth-13 Carboxylate | 0,5% |
| Sodium Amygdalus Dulcis Extract | 0,3% |
| Polyquaternium-10 | 0,2% |
| Tetrasodium EDTA | 0,1% |
| Quaternium-15 | 0,1% |
| Citric Acid | ad pH 5,2 |
| Preservative, Parfüm, Dyes | q.s. |

**Tabelle 41: Shampoo**

| | |
|---|---|
| Water | ad 100% |
| Cocamidopropyl Betaine | 4,0% |
| Sodium Lauryl Sulfoacetate | 3,0% |
| Produktbeispiel 1b: Sophorolipid + 10% Ölsäure | 2,0% |
| Disodium Laureth Sulfosuccinate | 1,0% |
| Sodium Lauroyl Sarcosinate | 1,0% |
| Glycol Distearate | 0,9% |
| Sodium Chloride | 0,9% |
| Decyl Glucoside | 0,5% |
| Polyquaternium-10 | 0,3% |
| PPG-5-Ceteth-20 | 0,5% |
| Coco-Betaine | 0,5% |
| PEG-55 Propylene Glycol Oleate | 0,4% |
| Propylene Glycol | 0,3% |
| Salicylic Acid | 0,2% |
| Carbomer | 0,3% |
| Sodium Hydroxide | 0,2% |
| Citric Acid | ad pH 5,5 |
| Preservative, Parfüm, Dyes | q.s. |

### Amphoacetat-basierte Systeme:

**Tabelle 42: Körperreinigungsmittel, PEG- & Sulfat-frei**

| | |
|---|---|
| Water | ad 100% |
| Sodium Cocoamphoacetate | 5,0% |
| Disodium Lauryl Sulfosuccinate | 1,2% |
| Produktbeispiel 1b: Sophorolipid + 10% Ölsäure | 2,5% |
| Cocamidopropyl Betaine | 3,0% |
| Cocamidopropyl Betaine (and) Glyceryl Laurate | 1,0% |
| Citric Acid, 30% | ad pH 5,0 |
| Preservative, Parfum | q.s. |

**Tabelle 43: Shampoo, PEG- & Sulfat-frei**

| | |
|---|---|
| Water | ad 100% |
| Sodium Cocoamphoacetate | 5,5% |
| Disodium Cocoyl Glutamate | 2,0% |
| Produktbeispiel 2b: Rhamnolipid + 10% Ölsäure | 2,0% |
| Polyquaternium-10 | 0,2% |
| Palmitamidopropyltrimonium Chloride | 1,0% |
| Isostearamide MIPA | 1,0% |
| Citric Acid, 30% | ad pH 5,5 |
| Preservative, Parfum | q.s. |

**Tabelle 44: Haar- & Körperreinigungsmittel, PEG- und Sulfat-frei**

| | |
|---|---|
| Sodium Cocoamphoacetate | 3,5% |
| Produktbeispiel 2b: Rhamnolipid + 10% Ölsäure | 3,5% |
| Disodium Lauryl Sulfosuccinate | 1,5% |
| Sorbitan Sesquicaprylate | 0,9% |
| Water | ad 100% |
| Cocamidopropyl Betaine | 3,0% |
| Citric Acid, 30% | ad pH 5,5 |
| Preservative, Parfum | q.s. |

**Tabelle 45: Mildes Gesichtsreinigungsgel, PEG- und Sulfat-frei, Ecocert-konform**

| | |
|---|---|
| Water | ad 100% |
| Xanthan Gum | 1,5% |
| Sodium Cocoamphoacetate | 3,0% |
| Produktbeispiel 1b: Sophorolipid + 10% Ölsäure | 2,5% |
| Cocamidopropyl Betaine | 2,0% |
| Capryl/Capramidopropyl Betaine | 2,0% |
| Sucrose Cocoate | 0,6% |
| Polyglyceryl-3 Caprate | 3,0% |
| Cocamidopropyl Betaine (and) Glyceryl Laurate | 1,3% |
| NaCl | 1,5% |
| Citric Acid, 30% | ad pH 5,0 |
| Preservative, Parfum | q.s. |

**Tabelle 46: Körperreinigungsmittel**

| | |
|---|---|
| Water | ad 100% |
| Glycerin | 5,0% |
| Helianthus Annuus Hybrid Oil | 3,5% |
| Cocamidopropyl Betaine | 3,5% |
| Sodium Hydroxypropyl Starch Phosphate | 3,0% |
| Sodium Laureth Sulfate | 3,0% |
| Sodium Cocoyl Glycinate | 2,5% |
| Produktbeispiel 2b: Rhamnolipid + 10% Ölsäure | 2,5% |
| Lauric Acid | 1,0% |
| Sodium Lauroyl Isethionate | 1,0% |
| Stearic Acid | 0,5% |
| Sodium Palmitate | 0,5% |
| Sodium Chloride | 0,5% |
| Guar Hydroxypropyltrimonium Chloride | 0,2% |
| Sodium Stearate | 0,2% |
| Tetrasodium EDTA | 0,1% |
| Tetrasodium Etidronate | 0,1% |
| Alumina | 0,1% |
| Citric Acid, 30% | ad pH 6,1 |
| Preservative, Parfüm, Dyes | q.s. |

### APG-basierte Systeme:

**Tabelle 47: Mildes Haar- und Körperreinigungsmittel**

| | |
|---|---|
| Water | ad 100% |
| Lauryl Glucoside | 5,0% |
| Coco Glucoside | 2,0% |
| Produktbeispiel 2b: Rhamnolipid + 10% Ölsäure | 3,0% |
| Sucrose Cocoate | 1,5% |
| Cocamidopropyl Betaine | 4,0% |
| Carbomer | 1,0% |
| Citric Acid, 30% | ad pH 5,5 |
| Preservative, Parfum | q.s. |

**Tabelle 48: Verwöhndusche**

| | |
|---|---|
| Water | ad 100% |
| Sesamum Indicum (Sesame) Seed Oil | 5,0% |
| Coco Glucoside | 5,0% |
| Alcohol | 5,0% |
| Produktbeispiel 2b: Rhamnolipid + 10% Ölsäure | 3,0% |
| Disodium Cocoyl Glutamate | 3,0% |
| Glycerin | 2,5% |
| Rosa Moschata Seed Oil | 0,5% |
| Xanthan Gum | 0,7% |
| Lactic Acid, 90% | ad pH 5,5 |
| Parfum | q.s. |

**Tabelle 49: Dusch- & Badegel**

| | |
|---|---|
| Water | ad 100% |
| Glycerin | 6,0% |
| Coco Glucoside | 5,5% |
| Caprylyl/Capryl Glucoside | 5,0% |
| Produktbeispiel 1b: Sophorolipid + 10% Ölsäure | 2,0% |
| Sodium Coco-Sulfate | 3,5% |
| Prunus Armeniaca (Apricot) Fruit Extract | 2,0% |
| Xanthan Gum | 2,0% |
| Alcohol | 1,0% |
| Parfum | q.s. |

**Tabelle 50: Duschgel**

| | |
|---|---|
| Water | ad 100% |
| Coco Glucoside | 7,0% |
| Sodium Coco Sulfate | 4,0% |
| Lauryl Glucoside | 2,0% |
| Produktbeispiel 1b: Sophorolipid + 10% Ölsäure | 2,0% |
| Glyceryl Oleate | 2,0% |
| Glycerin | 2,0% |
| Inulin | 0,5% |
| Aloe Barbadensis Leaf Juice | 0,4% |
| Sorbitol | 0,4% |
| Betaine | 0,2% |
| Sodium Cocoyl Glutamate | 0,2% |
| Disodium Cocoyl Glutamate | 0,2% |
| Cocos Nucifera (Coconut) Fruit Extract | 0,1% |
| Maris Sal | 0,1% |
| Glyceryl Caprylate | 0,1% |
| Sodium PCA | 0,1% |
| PCA Ethyl Cocoyl Arginate | 0,1% |
| Potassium Sorbate | 0,9% |
| Citric Acid | ad pH 5,5 |
| Parfum | q.s. |

**Tabelle 51: Kids Schaumbad**

| | |
|---|---|
| Water | ad 100% |
| Produktbeispiel 1b: Sophorolipid + 10% Ölsäure | 4,0% |
| Lauryl Glucoside | 4,0% |
| Aloe Barbadensis Leaf Extract | 4,0% |
| Sodium Lauryl Sulfoacetate | 2,0% |
| Glycerin | 2,0% |
| Polyglyceryl-10 Laurate | 2,0% |
| Coco Glucoside | 2,0% |
| Glyceryl Oleate | 0,5% |
| Disodium Cocoyl Glutamate | 0,4% |
| Sodium Cocoyl Glutamate | 0,4% |
| Calendula Officinalis Flower Extract | 0,2% |
| Sodium Chloride | 0,2% |
| Citric Acid | ad pH 5,5 |
| Parfum | q.s. |

**Tabelle 52: Duschgel**

| | |
|---|---|
| Water | ad 100% |
| Produktbeispiel 2b: Rhamnolipid + 10% Ölsäure | 4,0% |
| Coco Glucoside | 4,0% |
| Glycerin | 3,0% |
| Disodium Cocoyl Glutamate | 2,0% |
| Sodium Cocoyl Glutamate | 2,0% |
| Polyglyceryl-10 Laurate | 1,0% |
| Glyceryl Caprylate | 1,0% |
| Rubus Idaeus (Raspberry) Fruit Extract | 0,3% |
| Sodium PCA | 0,2% |
| Xanthan Gum | 0,6% |
| Glyceryl Oleate | 0,3% |
| Phytic Acid | 0,1% |
| Citric Acid | ad pH 5,5 |
| Parfum | q.s. |

**Tabelle 53: Ultra Sensitive Duschcreme**

| | |
|---|---|
| Water Aqua | ad 100% |
| Coco Glucoside | 7,0% |
| Alcohol | 3,0% |
| Produktbeispiel 1b: Sophorolipid + 10% Ölsäure | 3,0% |
| Glycerin | 2,5% |
| Simmondsia Chinensis Seed Oil | 1,5% |
| Xanthan Gum | 1,3% |
| Disodium Cocoyl Glutamate | 1,0% |
| Glyceryl Oleate | 0,8% |
| Sodium Cocoyl Glutamate | 0,5% |
| Citric Acid | ad pH 5,5 |
| Preservative, Parfum | q.s. |

**Tabelle 54: Anti-Schuppenshampoo**

| | |
|---|---|
| Water | ad 100% |
| Sorbitol | 5,5% |
| Glycerin | 5,0% |
| Coco Glucoside | 4,5% |
| Produktbeispiel 1b: Sophorolipid + 10% Ölsäure | 3,5% |
| Loess | 1,0% |
| Zea Mays Oil | 1,0% |
| Sodium Lauroyl Lactylate | 1,5% |
| Sodium Cocoyl Glutamate | 1,0% |
| Disodium Cocoyl Glutamate | 1,0% |
| Xanthan Gum | 0,7% |
| Kaolin | 0,3% |
| Canola Oil | 0,3% |
| Sodium Coco-Glucoside Tartrate | 0,2% |
| Tocopherol | 0,1% |
| Citric Acid | ad pH 5,3 |
| Preservative, Parfum | q.s. |

**Tabelle 55: Mildes Haar- & Körperreinigungsmittel, ECOCERT Inhaltsstoffe**

| | |
|---|---|
| Lauryl Glucoside | 3,0% |
| Produktbeispiel 1b: Sophorolipid + 10% Ölsäure | 2,0% |
| Cocamidopropyl Betaine (and) Glyceryl Laurate | 5,0% |
| Sorbitan Sesquicaprylate | 0,9% |
| Water | ad 100% |
| Cocamidopropyl Betaine | 4,0% |
| Citric Acid, 30 % | ad pH 5,5 |
| Preservative, Parfum | q.s. |

**Tabelle 56: Milder Reinigungsschaum**

| | |
|---|---|
| Water | ad 100% |
| Glycine Soja Oil | 8,0% |
| Glycerin | 5,0% |
| Alcohol | 5,0% |
| Produktbeispiel 2b: Rhamnolipid + 10% Ölsäure | 4,0% |
| Coco Glucoside | 3,5% |
| Caprylic/Capric Triglyceride | 2,0% |
| Sodium Coco Sulfate | 2,0% |
| Sodium Lactate | 1,0% |
| Sodium Cocoyl Glutamate | 1,0% |
| Disodium Cocoyl Glutamate | 0,6% |
| Argania Spinosa Kernel Oil | 0,6% |
| Preservative, Parfum | q.s. |

**Tabelle 57: Waschgel**

| | |
|---|---|
| Water | ad 100% |
| Alcohol | 7,0% |
| Glycerin | 5,5% |
| Coco Glucoside | 4,0% |
| Produktbeispiel 1b: Sophorolipid + 10% Ölsäure | 3,0% |
| Sodium Cocoyl Glutamate | 2,0% |
| Xanthan Gum | 2,0% |
| Equisetum Arvense Leaf Extract | 1,0% |
| Disodium Cocoyl Glutamate | 0,4% |
| Citric Acid | ad pH 5,5 |
| Preservative, Parfum | q.s. |

**Tabelle 58: Mildes Haar- & Körperreinigungsmittel, PEG- und Sulfat-frei**

| | |
|---|---|
| Lauryl Glucoside | 3,5% |
| Produktbeispiel 1b: Sophorolipid + 10% Ölsäure | 2,0% |
| Isostearamide MIPA (and) Glyceryl Laurate | 1,0% |
| Water | ad 100% |
| Coco Glucoside | 1,0% |
| Sodium Cocoamphoacetate | 3,0% |
| Cocoamidopropyl Betaine | 3,5% |
| Citric Acid, 30% | ad pH 5,5 |
| Preservative, Parfum | q.s. |

**Tabelle 59: Feuchte Tücher**

| | |
|---|---|
| Water | ad 100% |
| Caprylyl/Capryl Glucoside | 3,0% |
| Produktbeispiel 2b: Rhamnolipid + 10% Ölsäure | 3,5% |
| Coco Glucoside | 2,5% |
| Polyaminopropyl Biguanide | 1,0% |
| Propylene Glycol | 1,0% |
| Simethicone | 0,5% |
| Aloe Barbadensis Extract | 0,3% |
| Citric Acid | ad pH 5,7 |
| Preservative, Parfum | q.s. |

**Tabelle 60: Bio-Duschbad**

| | |
|---|---|
| Water | ad 100% |
| Glycerin | 4,0% |
| Lauryl Glucoside | 4,0% |
| Produktbeispiel 2b: Rhamnolipid + 10% Ölsäure | 3,5% |
| Ammonium Lauryl Sulfate | 3,0% |
| Cocamidopropyl Betaine | 2,0% |
| Lauroyl Sarcosine | 1,0% |
| Hydroxypropyl Guar Hydroxypropyltrimonium Chloride | 0,3% |
| Alcohol | 0,3% |
| Preservative, Parfüm, Dyes | q.s. |

**Tabelle 61: Baby Shampoo**

| | |
|---|---|
| Water | ad 100% |
| Coco-Glucoside | 4,5% |
| Sodium Lauroamphoacetate | 3,5% |
| Sodium Laureth Sulfate | 2,5% |
| Produktbeispiel 1b: Sophorolipid + 10% Ölsäure | 2,5% |
| Citric Acid | ad pH 5,5 |
| Polysorbate 20 | 0,7% |
| PEG-80 Sorbitan Laurate | 0,5% |
| PEG-150 Distearate | 0,3% |
| Polyquaternium-10 | 0,3% |
| Butylene Glycol | 0,2% |
| Propylene Glycol | 0,2% |
| Lactic Acid | 0,2% |
| Glucose | 0,1% |
| Preservative, Parfüm, Dyes | q.s. |

**Tabelle 62: Mildes Körperreinigungsmittel**

| | |
|---|---|
| Water | ad 100% |
| Lauryl Glucoside | 3,5% |
| Sorbitan Sesquicaprylate | 0,6% |
| Coco Glucoside | 0,5% |
| Produktbeispiel 2b: Rhamnolipid + 10% Ölsäure | 1,5% |
| Disodium Lauryl Sulfosuccinate | 2,0% |
| Cocamidopropyl Betaine | 3,5% |
| Citric Acid, 30% | 1,5% |
| Preservative, Parfum | q.s. |

**Tabelle 63: Duschgel**

| | |
|---|---|
| Water | ad 100% |
| Lauryl Glucoside | 8,0% |
| Cocamidopropyl Betaine | 2,5% |
| Produktbeispiel 1b: Sophorolipid + 10% Ölsäure | 2,5% |
| Dimethicone Propyl PG-Betaine | 0,3% |
| PEG-7 Glyceryl Cocoate | 2,0% |
| PEG-18 Glyceryl Oleate/Cocoate | 2,0% |
| Preservative, Parfum | q.s. |

**Tabelle 64: Mildes Körperreinigungsmittel, Ecocert-konform, PEG-, Sulfat- und Betain-frei**

| | |
|---|---|
| Water | ad 100% |
| Lauryl Glucoside | 4,0% |
| Produktbeispiel 2b: Rhamnolipid + 10% Ölsäure | 0,9% |
| Sorbitan Sesquicaprylate | 0,6% |
| Coco Glucoside, Cognis | 1,0% |
| Sodium/Disodium Cocoyl Glutamate | 2,0% |
| Sodium Cocoamphoacetate | 3,0% |
| Citric Acid | ad pH 5,1 |
| Preservative, Parfum | q.s. |

### Sulfonat-basierte Systeme:

**Tabelle 65: Sulfat-freies Shampoo mit MHEC**

| | |
|---|---|
| Water | ad 100% |
| Sodium C14-16 Olefin Sulfonate | 4,0% |
| Produktbeispiel 2b: Rhamnolipid + 10% Ölsäure | 3,0% |
| Cocoamidopropyl Betaine | 3,0% |
| Methylhydroxyethylcellulose | 1,5% |
| Preservative, Parfum | q.s. |

**Tabelle 66: Öl-freies Akne Reinigungsmittel**

| | |
|---|---|
| Water | ad 100% |
| Sodium C14-16 Olefin Sulfonate | 4,0% |
| Cocamidopropyl Betaine | 3,0% |
| Produktbeispiel 1b: Sophorolipid + 10% Ölsäure | 2,5% |
| Sodium C12-15 Pareth-15 Sulfonate | 2,5% |
| Aloe Extract | 1,0% |
| Camomile Extract | 1,0% |
| Linoleamidopropyl PG-Dimonium Chloride Phosphate | 0,7% |
| Disodium-EDTA | 0,2% |
| Propylene Glycol | 0,3% |
| Salicylic Acid | 2,0% |
| Sodium Choride | 1,0% |
| Preservative, Parfüm, Dyes | q.s. |

**Tabelle 67: Wasch-Emulsion für empfindliche und problematische Haut**

| | |
|---|---|
| Water | ad 100% |
| Sodium C14-16 Olefin Sulfonate | 4,0% |
| Sodium Laureth Sulfate | 3,0% |
| Produktbeispiel 1b: Sophorolipid + 10% Ölsäure | 2,0% |
| Disodium Laureth Sulfosuccinate | 1,5% |
| Sodium Chloride | 2,0% |
| Laureth-2 | 1,0% |
| Panthenol | 0,5% |
| Glycol Distearate | 0,5% |
| Saccaride Isomerate | 0,2% |
| Allantoin | 0,2% |
| Niacinamide | 0,1% |
| Pyridoxine HCl | 0,1% |
| Glycine | 0,1% |
| Alanine | 0,1% |
| Lysine | 0,1% |
| Biotin | 0,1% |
| Glycerin | 0,5% |
| Sodium Lauroyl Glutamate | 0,5% |
| Sodium Citrate | 0,5% |
| Cocamidopropyl Betaine | 0,5% |
| Sorbitan Laurate | 0,4% |
| PEG-120 Methyl Glucose Dioleate | 0,3% |
| Preservative, Parfum | q.s. |

### Sulfosuccinat-basierte Systeme:

**Tabelle 68: Kids Dusche & Shampoo**

| | |
|---|---|
| Water | ad 100% |
| Disodium Laureth Sulfosuccinate | 4,5% |
| Produktbeispiel 2b: Rhamnolipid + 10% Ölsäure | 3,5% |
| Cocamidopropyl Hydroxysultaine | 2,5% |
| Cocamidopropyl Betaine | 1,5% |
| Decyl Glucoside | 1,0% |
| PEG-18 Glyceryl Oleate/Cocoate | 0,7% |
| Laureth-2 | 0,5% |
| Sodium Chloride | 0,5% |
| Panthenol | 0,2% |
| Niacinamide | 0,1% |
| Pyridoxan Hydrochloride | 0,1% |
| Polyquaternium-10 | 0,3% |
| Glycerin | 0,3% |
| Inulin | 0,1% |
| Citric Acid | ad pH 5,7 |
| Preservative, Parfüm, Dyes | q.s. |

**Tabelle 69: Mildes Baby Shampoo**

| | |
|---|---|
| Water | ad 100% |
| Disodium Laureth Sulfosuccinate | 3,5% |
| Produktbeispiel 1b: Sophorolipid + 10% Ölsäure | 3,5% |
| Isostearamide MIPA (and) Glyceryl Laurate | 1,7% |
| PEG-7 Glyceryl Cocoate | 0,5% |
| Sodium Cocoamphoacetate | 3,0% |
| Palmitamidopropyltrimonium Chloride | 2,3% |
| Citric Acid, 30% | ad pH 6,0 |
| Preservative, Parfüm, Dyes | q.s. |

**Tabelle 70: Milder Gesichtsreinigungsschaum**

| | |
|---|---|
| Water | ad 100% |
| Disodium PEG-5 Laurylcitrate Sulfosuccinate (and) Capryl/ Capramidopropyl Betaine | 5,5% |
| Produktbeispiel 1b: Sophorolipid + 10% Ölsäure | 3,0% |
| Capryl/Capramidopropyl Betaine | 2,0% |
| Polyglyceryl-3 Caprate | 0,5% |
| Creatine | 0,2% |
| Hydroxypropyl Methylcellulose | 0,5% |
| Sodium Lactate (and) Sodium PCA (and) Glycine (and) Fructose (and) Urea (and) Niacinamide (and) Inositol (and) Sodium Benzoate (and) Lactic Acid | 1,0% |
| Preservative, Parfum | q.s. |

### Sarcosinat-basierte Systeme:

**Tabelle 71: Shampoo**

| | |
|---|---|
| Water | ad 100% |
| Sodium Lauroyl Sarcosinate | 5,0% |
| Coco Betaine | 3,0% |
| Produktbeispiel 2b: Rhamnolipid + 10% Ölsäure | 1,5% |
| Cocamide DEA | 4,5% |
| Cetrimonium Chloride | 0,3% |
| Steralkonium Chloride | 0,1% |
| Disodium EDTA | 0,2% |
| Citric Acid | ad pH 6,7 |
| Preservative, Parfum | q.s. |

**Tabelle 72: Shampoo**

| | |
|---|---|
| Water | ad 100% |
| Sodium Lauroyl Sarcosinate | 5,0% |
| Produktbeispiel 1b: Sophorolipid + 10% Ölsäure | 2,5% |
| Cocamidopropyl Betaine | 4,0% |
| Palmitamidopropyltrimonium Chloride | 0,5% |
| Polyquaternium-10 | 0,1% |
| Citric Acid | ad pH 5,1 |
| Preservative, Parfum | q.s. |

**Tabelle 73: Shampoo**

| | |
|---|---|
| Water | ad 100% |
| Sodium Lauroyl Sarcosinate | 3,5% |
| Produktbeispiel 1b: Sophorolipid + 10% Ölsäure | 3,5% |
| Sodium Cocoamphoacetate | 2,5% |
| Cocamidopropyl Betaine | 1,0% |
| Coco-Betaine | 1,0% |
| Coco-Glucoside | 0,5% |
| Glyceryl Oleate | 0,5% |
| Polyquaternium-7 | 0,3% |
| Polysorbate 20 | 0,2% |
| Silicone Quaternium-22 | 0,3% |
| PEG-120 Methyl Glucose Dioleate | 0,3% |
| Hibiscus Sabdariffa (Hibiscus) Extract | 0,1% |
| Glycerin | 0,2% |
| Sodium Hydroxymethylglycinate | 0,2% |
| Citric Acid | ad pH 5,1 |
| Preservative, Parfum | q.s. |

**Tabelle 74: Mildes Körperreinigungsmittel**

| | |
|---|---|
| Water | ad 100% |
| Sodium Lauroyl Sarcosinate | 2,5% |
| Sodium Lauroamphoacetate | 2,5% |
| Sodium Cocoyl Alaninate | 2,0% |
| Produktbeispiel 2b: Rhamnolipid + 10% Ölsäure | 1,5% |
| Sodium Cocoamphoacetate | 1,5% |
| Lauryl Glucoside | 1,5% |
| Disodium Cocoyl Glutamate | 0,5% |
| Sodium Lauryl Glucose Carboxylate | 0,5% |
| PEG-150 Distearate | 0,5% |
| Polysorbate 20 | 0,4% |
| Glycerin | 0,5% |
| Algae Extract | 0,1% |
| Methyl Gluceth-20 | 0,2% |
| Coco-glucoside | 0,3% |
| Glyceryl Oleate | 0,5% |
| Allantoin | 0,1% |
| PEG-40 Hydrogenated Castor Oil | 0,4% |
| Disodium EDTA | 0,1% |
| Citric Acid | ad pH 5,5 |
| Preservative, Parfum | q.s. |

### anders-basierte Systeme (Sultain, Anisat, Isethionat, Glutamat, Glycinat):

**Tabelle 75: Baby Pflegebad**

| | |
|---|---|
| Water | ad 100% |
| Sorbitol | 3,5% |
| Cocamidopropyl Hydroxysultaine | 3,5% |
| Produktbeispiel 1b: Sophorolipid + 10% Ölsäure | 3,0% |
| Cocamidopropyl Betaine | 2,5% |
| Lauryl Glucoside | 1,0% |
| Coco-Glucoside | 1,0% |
| Sodium Chloride | 1,5% |
| Glyceryl Oleate | 0,9% |
| Sodium Amygdalus Dulcis Extract | 0,3% |
| Sodium Carboxymethyl Betaglucan | 0,3% |
| Glycerin | 0,3% |
| Styrene/Acrylates Copolymer | 0,3% |
| Inulin | 0,1% |
| Preservative, Parfüm, Dyes | q.s. |

**Tabelle 76: Feuchte Reinigungstücher**

| | |
|---|---|
| Water | ad 100% |
| Glycerin | 5,0% |
| Sodium Anisate | 3,5% |
| Sodium Levulinate | 3,5% |
| Produktbeispiel 1b: Sophorolipid + 10% Ölsäure | 3,0% |
| Glyceryl Caprylate | 1,0% |
| Tocopheryl Acetate | 0,5% |
| Sodium Citrate | 0,5% |
| Xanthan Gum | 0,5% |
| Hydrolyzed Milk Protein | 0,4% |
| Prunus Armeniaca Kernel Oil | 0,3% |
| Magnesium Stearate | 0,2% |
| Preservative, Parfum | q.s. |

**Tabelle 77: Duschgel**

| | |
|---|---|
| Water | ad 100% |
| Sodium Lauroyl Methyl Isethionate | 4,5% |
| Produktbeispiel 1b: Sophorolipid + 10% Ölsäure | 3,0% |
| Cocamidopropyl Betaine | 2,5% |
| Sodium Chloride | 2,5% |
| Glycerin | 1,5% |
| Polyglyceryl-4 Caprate | 0,6% |
| Sucrose Cocoate | 0,5% |
| Trisodium Ethylenediamine Disuccinate | 0,2% |
| Zinc Laurate | 0,1% |
| Salicylic Acid | 0,1% |
| Propylene Glycol | 0,1% |
| Aloe Barbadensis Leaf Juice | 0,1% |
| Sodium Hydroxide | 0,1% |
| Tocopherol | 0,1% |
| Citric Acid | ad pH 5,5 |
| Preservative, Parfum | q.s. |

**Tabelle 78: Shampoo**

| | |
|---|---|
| Water | ad 100% |
| Sodium Cocoyl Isethionate | 3,0% |
| Produktbeispiel 2b: Rhamnolipid + 10% Ölsäure | 3,0% |
| Cocamidopropyl Hydroxysultaine | 3,0% |
| Cocamide DEA | 2,5% |
| Sodium Lauroyl Methyl Isethionate | 2,0% |
| Inulin | 1,0% |
| Sodium Cocoyl Glutamate | 0,7% |
| Sodium Lauroyl Glutamate | 0,7% |
| Hydrolyzed Wheat Protein | 0,4% |
| Hydroxypropyl Guar Hydroxypropyltrimonium Chloride | 0,1% |
| Polyquaternium-10 | 0,1% |
| Arnica Montana Flower Extract | 0,1% |
| Styrene/Acrylates Copolymer | 0,2% |
| Disodium EDTA | 0,1% |
| Sodium Hydroxide | 0,1% |
| Lactic Acid, 90% | ad pH 5,5 |
| Preservative, Parfum | q.s. |

**Tabelle 79: Babyreinigungsmittel**

| | |
|---|---|
| Water | ad 100% |
| Glycerin | 4,5% |
| Produktbeispiel 1b: Sophorolipid + 10% Ölsäure | 3,5% |
| Pentylene Glycol | 3,0% |
| Sodium Cocoamphoacetate | 3,0% |
| Cocamidopropyl Betaine | 3,0% |
| Helianthus Annuus Seed Oil | 1,6% |
| Lauryl Glucoside | 1,5% |
| Sodium PEG-7 Olive Oil Carboxylate | 0,5% |
| Hydroxyethylcellulose | 0,5% |
| Sodium Cocoyl Glutamate | 0,5% |
| Sodium Lauryl Glucose Carboxylate | 0,5% |
| Sodium Cocoa Butter Amphoacetate | 0,3% |
| Preservative, Parfum | q.s. |

**Tabelle 80: Körper- und Haareinigungsschaum**

| | |
|---|---|
| Water | ad 100% |
| Sodium Cocoyl Glutamate | 5,0% |
| Glycerin | 3,0% |
| Sodium Laureth Sulfate | 3,0% |
| Produktbeispiel 2b: Rhamnolipid + 10% Ölsäure | 2,0% |
| Pentylene Glycol | 2,0% |
| Sodium Cocoyl Alaninate | 1,0% |
| PEG-200 Hydrogenated Glyceryl Palmate (and) PEG-7 Glyceryl Cocoate | 1,2% |
| PEG-60 Glyceryl Isostearate | 0,5% |
| Cocamidopropyl PG-dimonium Chloride Phosphate | 0,3% |
| Butylene Glycol | 0,3% |
| Propylene Glycol | 0,5% |
| Ginkgo Biloba Leaf Extract | 0,1% |
| Tetrasodium EDTA | 0,1% |
| Benzophenone-4 | 0,2% |
| Citric Acid | ad pH 5,3 |
| Preservative, Parfum, Dyes | q.s. |

**Tabelle 81: Make-Up Entferner**

| | |
|---|---|
| Water | ad 100% |
| PEG-8 | 5,0% |
| Isopentyldiol | 2,0% |
| PEG-20 Glyceryl Triisostearate | 2,0% |
| Glycerin | 2,0% |
| Hydrogenated Polyisobutene | 2,0% |
| Potassium Cocoyl Glycinate | 2,0% |
| Lauryl Hydroxysultaine | 2,0% |
| Produktbeispiel 1b: Sophorolipid + 10% Ölsäure | 2,0% |
| Butylene Glycol Laurate | 1,5% |
| Lauryl Glucoside | 1,5% |
| Alcohol | 0,5% |
| Disodium EDTA | 0,1% |
| Citric Acid | ad pH 5,5 |
| Preservative, Parfum, Dyes | q.s. |

**Tabelle 82: Schäumendes Körperreinigungsmittel**

| | |
|---|---|
| Water | ad 100% |
| Sodium Cocoyl Glycinate | 6,0% |
| Produktbeispiel 2b: Rhamnolipid + 10% Ölsäure | 4,0% |
| Coco-betaine | 2,0% |
| Glycerin | 1,0% |
| Sodium Chloride | 1,0% |
| Acrylates/C10-30 Alkyl Acrylate Crosspolymer | 0,5% |
| Sodium Hydroxide | 0,4% |
| PEG-14M | 0,3% |
| Salicylic Acid | 0,1% |
| Polyquaternium-10 | 0,1% |
| Glycol Distearate | 0,2% |
| Citric Acid | ad pH 5,5 |
| Preservative, Parfum, Dyes | q.s. |

**Tabelle 83: Weitere Formulierungsbeispiele**

| | **I** | **II** | **III** | **IV** | **V** | **VI** | **VII** | **VIII** | **IX** | **X** |
|---|---|---|---|---|---|---|---|---|---|---|
| **Wasser** | ad 100% | | | | | | | | | |
| **Produktbeispiel 1b: Sophorolipid + 10% Ölsäure** | 10,0% | - | 3,0% | 3,5% | - | 20% | - | 4,0% | - | 3,0% |
| **Produktbeispiel 2b: Rhamnolipid + 10% Ölsäure** | - | 12,0% | 5,5% | - | 7,0% | 2,0% | 5,5% | - | 3,5% | - |
| **Sodium Laureth Sulfate** | - | - | - | 7,0% | - | - | - | 4,5% | - | - |
| **Ammonium Lauryl Sulfate** | - | - | - | - | 5,0% | - | - | - | 3,5% | - |
| **Cocamidopropyl Betaine** | - | - | - | - | - | 6,0% | - | 2,5% | - | 4,5% |
| **Sodium Cocoamphoacetate** | - | - | - | - | - | - | - | - | 3,5% | - |
| **Coco Glucoside** | - | - | - | - | - | - | 5,5% | - | - | 2,5% |
| **Sucrose Cocoate** | 0,5% | 1,0% | 1,0% | 1,0% | 1,0% | 0,5% | 1,0% | 1,0% | 1,0% | 1,0% |
| **Hydroxypropyl Methylcellulose** | 0,5% | - | - | - | - | 0,3% | - | - | - | 0,1% |
| **Polyglyceryl-4 Caprate** | - | - | 1,0% | - | - | - | - | - | - | - |
| **Hydroxypropyl Guar Hydroxypropyltrimonium Chloride** | 0,2% | 0,2% | 0,2% | 0,2% | 0,2% | 0,2% | 0,2% | 0,2% | 0,2% | 0,2% |
| **Silicone Quaternium-22** | - | - | - | 0,5% | 0,5% | - | - | - | - | - |
| **Amodimethicone** | - | - | - | - | - | - | 0,5% | - | - | - |
| **Glycol Distearate** | - | - | 0,5% | - | 0,5% | - | - | - | 0,5% | 0,5% |
| **Carbomer** | 1,0% | - | - | - | 0,5% | - | - | - | 0,5% | - |
| **Sodium Hydroxide, 25%** | 1,5% | - | - | - | 0,8% | - | - | - | 07% | - |
| **Isostearamide MIPA; Glyceryl Laurate** | - | 1,5% | - | - | - | - | - | - | 1,0% | 0,5% |
| **Sorbitan Sesquicaprylate** | - | 0,5% | - | 0,5% | 0,5% | 1,2% | 1,0% | - | - | 1,3% |
| **Sodium Chloride** | 1,0% | 1,0% | - | 0,5% | - | 1,5% | - | 2,0% | 1,5% | - |
| **PEG-200 Hydrogenated Glyceryl Palmate; PEG-7 Glyceryl Cocoate** | - | - | - | 0,4% | - | - | - | 0,6% | - | - |
| **Xanthan Gum** | - | - | 1,2% | - | - | - | 1,0% | - | - | 0,5% |
| **Panthenol** | 0,1% | 0,1% | 0,1% | 0,1% | 0,1% | 0,1% | 0,1% | 0,1% | 0,1% | 0,1% |
| **Citric Acid** | ad pH 5,5 | | | | | | | | | |
| **Preservative, Parfum, Dyes** | q.s. | | | | | | | | | |

**Tabelle 84: Weitere Formulierungsbeispiele**

| | **XI** | **XII** | **XIII** | **XIV** | **XV** | **XVI** | **XVII** | **XVIII** | **XIX** | **XX** |
|---|---|---|---|---|---|---|---|---|---|---|
| **Wasser** | ad 100% | | | | | | | | | |
| **Produktbeispiel 1b: Sophorolipid + 10% Ölsäure** | 8,0% | - | 9,0% | 3,0% | - | 5,0% | - | 6,5% | 1,7% | 1,5% |
| **Produktbeispiel 2b: Rhamnolipid + 10% Ölsäure** | - | 7,0% | - | 4,0% | 7,0% | - | 5,5% | - | 0,5% | - |
| **Disodium Lauryl Sulfosuccinate** | 5,0% | - | - | - | - | 2,0% | - | - | - | - |
| **Sodium Lauryl Sulfate** | - | - | - | - | - | - | - | - | - | 6,0% |
| **Cocamidopropyl Betaine** | - | 5,0% | - | - | - | - | 2,5% | - | 6,0% | 2,5% |
| **Coco-Betaine** | - | - | - | 2,0% | - | - | 2,0% | - | 1,0% | - |
| **Lauryl Glucoside** | - | - | - | - | 3,0% | 2,0% | - | 4,5% | 4,5% | - |
| **Sodium Cocoyl Glutamate** | - | - | 3,5% | - | 1,5% | 1,0% | - | - | - | - |
| **Sodium Lauroyl Sarcosinate** | - | - | - | 0,9% | - | 1,0% | 2,5% | - | - | - |
| **Sodium Cocoyl Isethionate** | - | - | - | - | - | - | - | - | - | 1.0% |
| **Sucrose Cocoate** | 0,5% | 1,0% | 0,3% | 1,0% | - | - | 1,0% | 1,0% | 0,1% | 0,5% |
| **Polyglyceryl-3 Caprate** | - | - | 1,0% | - | - | - | - | - | - | - |
| **Alcohol** | - | - | - | - | 0,3% | 0,1% | - | - | - | - |
| **Glycerin** | - | - | 0,5% | - | 5,0% | 2,0% | - | 3,5% | - | - |
| **Polyquaternium-10** | 0,2% | 0,2% | 0,2% | 0,2% | 0,2% | 0,2% | 0,2% | 0,3% | 0,3% | 0,2% |
| **Dimethicone** | - | - | - | - | - | - | 0,5% | - | - | - |
| **Hydrolyzed Wheat Protein** | 0,2% | - | 0,1% | - | 0,1% | - | - | - | 0,1% | - |
| **Glycol Distearate** | - | - | 0,5% | - | 0,3% | - | 0,5% | - | - | - |
| **Carbomer** | 1,0% | - | - | - | 0,5% | - | - | - | - | - |
| **Sodium Hydroxide, 10%** | q.s. | - | - | - | 2,5% | - | - | - | - | - |
| **Isostearamide MIPA; Glyceryl Laurate** | - | 1,5% | - | - | - | - | - | - | 1,5% | 1,2% |
| **Cocamide DEA** | - | 0,5% | - | 0,5% | - | 1,2% | 2,0% | - | - | 0,3% |
| **Sodium Chloride** | 1,0% | 1,0% | - | 0,5% | - | 1,5% | - | 2,0% | 0,3% | 0,2% |
| **PEG-120 Methyl Glucose Dioleate** | 0,3% | - | - | - | - | - | - | - | - | - |
| **Xanthan Gum** | - | - | 0,9% | - | 1,0% | 0,3% | - | 1,0% | - | - |
| **Creatine** | 0,5% | 0,1% | 0,1% | 0,1% | 0,1% | 0,1% | 0,1% | 0,1% | 0,1% | 0,1% |
| **Citric Acid** | ad pH 5,2 | | | | | | | | | |
| **Preservative, Parfum, Dyes** | q.s. | | | | | | | | | |

**Tabelle 85: Liste der verwendeten Rohstoffe:**

| **INCI-Name:** | **Handelsname:** |
|---|---|
| **Fettalkohole:** | |
| Cetyl Alcohol | TEGO Alkanol 16, Evonik Industries AG, 100% |

| **Verdicker/Stabilisatoren :** | |
|---|---|
| Carbomer | TEGO Carbomer 140, Evonik Industries AG, 100% |
| Hydroxyethylcellulose | Natrosol Hydroxyethylcellulose, Ashland Aqualon |
| Acrylates / C10-30 Alkyl Acrylate Crosspolymer | TEGO Carbomer 341 ER, Evonik Industries AG, 100% |
| Cocamide DEA | REWOMID DC 212 S, Evonik Industries AG, 100% |
| Cocamide MEA | REWOMID D 212, Evonik Industries AG, 100% |
| Isostearamide MIPA; Glyceryl Laurate | ANTIL SPA 80, Evonik Industries AG, 100% |
| Sorbitan Sesquicaprylate | ANTIL Soft SC, Evonik Industries AG, 100% |
| Laureth-4 | TEGO Alkanol L 4, Evonik Industries AG, 100% |
| PEG-60 Glyceryl Isostearate | Emalex GWIS-160N, Nihon Emulsion Company |
| PEG-150 Distearate | REWOPAL PEG 6000 DS, Evonik Industries AG, 100% |
| PEG-120 Methyl Glucose Dioleate | ANTIL 120 Plus, Evonik Industries AG, 100% |
| PEG-18 Glyceryl Oleate/Cocoate | ANTIL 171, Evonik Industries AG, 100% |
| PEG-200 Hydrogenated Glyceryl Palmate; PEG-7 Glyceryl Cocoate | REWODERM LI S 80, Evonik Industries AG, 100% |
| Xanthan Gum | Keltrol CG-SFT, CP Kelco, 100% |

| **Perlglanzmittel:** | |
|---|---|
| PEG-3 Distearate | Cutina TS, BASF Cognis, 100% |
| Glycol Distearate | TEGIN G 1100, Evonik Industries AG, 100% |
| Glycol Distearate; Laureth-4, Cocamidopropyl Betaine | TEGO Pearl N 300, Evonik Industries AG |

| **Silicone:** | |
|---|---|
| Amodimethicone | DC 949, Dow Corning, 100% |
| Dimethicone | ABIL 350, Evonik Industries AG, 100% |
| Dimethicone (and) Dimethiconol | DC 1503. Dow Corning |
| Quaternium-80 | ABIL Quat 3272, Evonik Industries AG, 30% |
| Silicone Quaternium-22 | ABIL T Quat 60, Evonik Industries AG, 65% |
| Dimethicone Propyl PG-Betaine | ABIL B 9950, Evonik Industries AG, 30% |
| Methoxy PEG/PPG-7/3 Aminopropyl Dimethicone | ABIL Soft AF 100, Evonik Industries AG, 100% |
| Bis-PEG/PPG-20/20 Dimethicone | ABIL B 8832, Evonik Industries AG, 100% |
| PEG/PPG-14/4 Dimethicone | ABIL B 8851, Evonik Industries AG, 95% |

| **Pflegepolymere/Filmformer:** | |
|---|---|
| Polyquaternium-7 | Merquat 550, Nalco, 100% |
| Polyquaternium-10 | Polymer JR 400, Amerchol, 100% |
| Styrene/Acrylates Copolymer | Yodosol GH840, Akzo Nobel |
| Starch Hydroxypropyltrimonium Chloride | Sensomer CI-50, Nalco |
| Hydroxypropyl Guar Hydroxypropyltrimonium Chloride | Jaguar C-162, Rhodia, 100% |

| **Wirkstoffe:** | |
|---|---|
| Panthenol | D-Panthenol USP, BASF, 100% |
| Allantoin | Allantoin, DSM |
| Tocopheryl Acetate | Tinoderm E, BASF |
| Inulin | Inutec H25, Beneo-Remy |
| Niacinamide | Niacinamide, USP, DSM Nutrional Products, 100% |
| Creatine | TEGO Cosmo C 100, Evonik Industries AG, 100% |
| Sodium Lactate; Sodium PCA; Glycine; Fructose; Urea; Niacinamide; Inositol; Sodium Benzoate; Lactic Acid | LACTIL, Evonik Industries AG, 100% |
| Salicylic Acid | AEC Salicylic Acid, A & E Connock |
| Hydrolyzed Wheat Protein | Gluadin WLM, BASF Cognis |

| **Anti-Schuppen-Wirkstoffe:** | |
|---|---|
| Climbazol | Crinipan AD, Haarmann & Reimer Fragrance GmbH, 100% |
| Octopirox | Octopirox, Clariant |
| Zinc Pyrithione | Zinc-Pyrion NF, WeylChem, 48% |

| **Konditioner:** | |
|---|---|
| Cetrimonium Chlorid | VARISOFT 300, Evonik Industries AG, 30% |
| Palmitamidopropyltrimonium Chloride | VARISOFT PATC, Evonik Industries AG, 60% |

| **Tenside:** | |
|---|---|
| Cocamidopropyl Betaine | TEGO Betain F 50, Evonik Industries AG, 38% |
| Cocamidopropyl Betaine | TEGO Betain CK D, Evonik Industries AG, 82% |
| Capryl/Capramidopropyl Betaine | TEGO Betain 810, Evonik Industries AG, 35% |
| Undecylenamidopropyl Betaine | REWOTERIC AM B U 185, Evonik Industries AG, 30% |
| Coco-Betaine | Dehyton AB 30, BASF Cognis, 31% |
| Sodium Cocoamphoacetate | REWOTERIC AM C, Evonik Industries AG, 32% |
| Sodium Cocoamphopropionate | REWOTERIC AM KSF 40, Evonik Industries AG, 40% |
| Disodium Ricinoleamido MEA-Sulfosuccinate | REWODERM S 1333, Evonik Industries AG, 40% |
| Disodium Lauryl Sulfosuccinate | REWOPOL SB F 12 P, Evonik Industries AG, 95% |
| Disodium Laureth Sulfosuccinate | REWOPOL SB FA 30 B, Evonik Industries AG, 40% |
| Disodium PEG-5 Laurylcitrate Sulfosuccinate; Capryl/Capramidopropyl Betaine | REWOPOL SB C 55, Evonik Industries AG, 54% |
| Sodium Laureth Sulfate | Texapon NSO, BASF Cognis, 28% |
| Sodium Lauryl Sulfate | Texapon LS 35, BASF Cognis, 30% |
| Sodium Myreth Sulfate | Texapon K 14S Spez , BASF Cognis, 70% |
| Ammonium Laureth Sulfate | Standapol EA-1, BASF Cognis |
| Sodium Coco-Sulfate | Texapon HC G, BASF Cognis |
| Sodium Trideceth Sulfate | Rhodapex EST-30, Rhodia |
| Ammonium Lauryl Sulfate | Standapol A, BASF Cognis |
| MIPA-Laureth Sulfate | Zetesol 2056, Zschimmer & Schwarz GmbH |
| Hydroxysultaine | OriStar HSB, Orient Stars LLC |
| Cocamidopropyl Hydroxysultaine | Mirataine CBS, Rhodia |
| Lauryl Glucoside | Plantacare 1200 UP, BASF Cognis, 50% |
| Decyl Glucoside | Plantacare 2000 UP, BASF Cognis |
| Coco Glucoside | Plantacare 818 UP, BASF Cognis, 51% |
| Caprylyl/Capryl Glucoside | Plantacare 810 UP, BASF Cognis |
| Glyceryl Glucoside | Hydagen GG, BASF Cognis |
| Sodium Cocoyl Glutamate | Plantapon ACG HC, BASF Cognis |
| Disodium Cocoyl Glutamate | Planatpon ACG LC, BASF Cognis |
| Sodium Cocoyl Hydrolyzed Wheat Protein Glutamate | PGFAC-S, BASF Cognis |
| Lauroamphoglycinate | Miranol Ultra L-32, Rhodia |
| Sodium Cocoyl Glycinate | Hostapon SG, Clariant |
| Sodium Lauryl Sulfoacetate; Disodium Laureth Sulfosuccinate | Stepan-Mild LSB, Stepan |
| Sodium C14-16 Olefin Sulfonate | Bioterge AS-40 AOS, Stepan |
| Sodium C12-15 Pareth-15 Sulfonate | Avanel S-150 CGN, BASF |
| Sodium Anisate; Sodium Levulinate | Dermosoft 1388, Dr. Straetmans |
| Sodium Coco-Glucoside Tartrate | Eucarol AGE/ET, Cesalpina Chemicals S.P.A. |
| Sodium Lauroyl Lactylate | Dermosoft SLL, Dr. Straetmans |
| Sodium Lauroyl Sarcosinate | Crodasinic LS 30, Croda, 30% |
| Sodium Cocoyl Sarcosinate | Crodasinic CS, Croda |
| Sodium Myristoyl Sarcosinate | Crodasinic MS, Croda |
| Lauroyl Sarcosine | Crodasinic L, Croda |
| C12-14 Hydroxyalkyl Hydroxyethyl Sarcosine | Softazoline LMEB, Kawaken Fine Chemical Co. |
| Sodium Methyl Cocoyl Taurate | Adinol CT, Croda |
| Sodium Cocoyl Alaninate | Amilite ACS-12, Ajinomoto |
| Sodium Lauryl Glucose Carboxylate | Plantapon SF, BASF Cognis |
| Sodium Cocoabutteramphoacetate | Mackam 1CB, Rhodia |
| Cocamidopropyl PG-dimonium Chloride Phosphate | Arlasilk PTC, Croda |
| Sodium Lauroyl Methyl Isethionate | Iselux, Innospec Active Chemicals |
| Sodium Cocoyl Isethionate | Hostapon STCI-85 P, Clariant |

| **Rückfetter / Emollients:** | |
|---|---|
| Polyglyceryl-3 Caprate | TEGOSOFT PC 31, Evonik Industries AG, 100% |
| Glyceryl Caprylate | Dermosoft GMCY, Dr. Straetmans |
| Glyceryl Stearate | TEGIN 4100 Pellets, Evonik Industries AG, 100% |
| Glyceryl Oleate | TEGIN O V, Evonik Industries AG, 100% |
| Diethylhexyl Carbonate | TEGOSOFT DEC, Evonik Industries AG, 100% |
| Caprylic/Capric Triglyceride | TEGOSOFT CT, Evonik Industries AG, 100% |
| PEG-7 Glyceryl Cocoate | TEGOSOFT GC, Evonik Industries AG, 100% |
| Sucrose Cocoate | TEGOSOFT LSE 65 K, Evonik Industries AG, 100% |
| PEG-3 Myristyl Ether | TEGOSOFT APM, Evonik Industries AG, 100% |
| Sorbitol | Sorbitol USP Powder, Lipo |
| Glycerin | Glycerol EP, vegetable, Spiga Nord, 99,7% |
| Hydrogenated Didecene | Satin Touch HD 2, Chevron Phillips |
| Dicaprylylether | Cetiol OE, BASF Cognis |

| **Schaum booster:** | |
|---|---|
| Hydroxypropyl Methylcellulose | TEGOCEL HPM 50, Evonik Industries AG, 100% |

| **Solubilisatoren:** | |
|---|---|
| PEG-40 Hydrogenated Castor Oil | TAGAT CH 40, Evonik Industries AG, 100% |
| PEG-20 Glyceryl Laurate | TAGAT L 2, Evonik Industries AG, 100% |
| Pentylene Glycol | Hydrolite-5 616751, Symrise |
| Polysorbate 20 | TEGO SML 20, Evonik Industries AG, 100% |
| Polyglyceryl-4 Caprate | TEGOSOFT PC-41, Evonik Industries AG, 100% |

## Patentansprüche

1. Zusammensetzung enthaltend Wasser,
mindestens ein Biotensid ausgewählt aus Rhamnolipiden und Sophorolipiden und mindestens eine Fettsäure ausgewählt aus Heptansäure, Octansäure, Pelargonsäure, Decansäure, Dodecansäure, Tetradecansäure, Palmitinsäure, Margarinsäure, Stearinsäure, Arachinsäure, Behensäure, Tetracosansäure, Cerotinsäure, Triacontansäure, Tubercolostearinsäure, Ölsäure, Elaidinsäure, Erucasäure, Linolsäure, Linolensäure, Elaeostearinsäure, Arachidonsäure, Eicosapentaensäure und Clupanodonsäure,
**dadurch gekennzeichnet, dass** der Anteil der Summe aller Tenside an der Zusammensetzung von 5 bis 30 Gew.-% beträgt, und dass der Anteil Fettsäure bezogen auf die Summe aus Fettsäure und Tensiden von 0,1 bis 20 Gew.-% beträgt, **dadurch gekennzeichnet, dass** die Zusammensetzung mindestens ein Verdickungsmittel enthält,
wobei mindestens 90 Gew.-% der Verdickungsmittel ausgewählt sind aus Xanthan-Gum, Guar, Agar-Agar, Alginate, Tylosen, Cellulose, welche teilweise oder vollständig auf nachwachsenden Rohstoffen basieren.

2. Zusammensetzung nach Anspruch 1, **dadurch gekennzeichnet, dass** der Anteil an Verdickungsmitteln an der Zusammensetzung von 0,1 bis 10 Gew.-% bezogen auf die Zusammensetzung beträgt.

3. Zusammensetzung nach zumindest einem der Ansprüche 1 oder 2, **dadurch gekennzeichnet, dass** die Zusammensetzung außerdem mindestens ein Tensid, welches kein Biotensid ist, enthält.

4. Zusammensetzung nach Anspruch 3, **dadurch gekennzeichnet, dass** das Gewichtsverhältnis von Biotensiden zu Tensiden, die kein Biotensid sind >1 zu 1 beträgt.

5. Zusammensetzung nach Anspruch 3, **dadurch gekennzeichnet, dass** das Gewichtsverhältnis von Biotensiden zu Tensiden, die kein Biotensid sind <=1 zu 1 beträgt.

6. Zusammensetzung nach einem der Ansprüche 1 bis 5, **dadurch gekennzeichnet, dass** die Zusammensetzung keine Tenside enthält, die Sulfate oder Polyethylenglykol aufweisen.

7. Zusammensetzung nach einem der Ansprüche 1 bis 6, **dadurch gekennzeichnet, dass** die Zusammensetzung einen oder mehrere Naturfarbstoffe aufweist.

8. Zusammensetzung nach einem der Ansprüche 1 bis 7, **dadurch gekennzeichnet, dass** die Zusammensetzung frei von Konservierungsmitteln gemäß INCI ist.

9. Zusammensetzung nach einem der Ansprüche 1 bis 8, **dadurch gekennzeichnet, dass** die Zusammensetzung Propylenglykol, Harnstoff, Glycerin, ätherische Öle, Phenylethylalkohol und/oder Ethanol enthält.

10. Zusammensetzung nach einem der Ansprüche 1 bis 9, **dadurch gekennzeichnet, dass** die Zusammensetzung als Biotenside Mono-, Di- oder Polyrhamnolipide und/oder Sophorolipide aufweist.

11. Zusammensetzung nach einem der Ansprüche 1 bis 10, **dadurch gekennzeichnet, dass** die Zusammensetzung Parfümöle oder Duftstoffe aufweist, wobei der Anteil der natürlichen Riechstoffe bezogen auf die Gesamtzahl der Riechstoffe in den Parfümölen mindestens 50 Gew.-% beträgt.

12. Zusammensetzung nach einem der Ansprüche 1 bis 11, **dadurch gekennzeichnet, dass** die Zusammensetzung ein Körper-, Haut- oder Haarbehandlungsmittel ist.

13. Verwendung einer Zusammensetzung nach einem der Ansprüche 1 bis 12 als oder zur Herstellung von Badezusätzen, Duschgel, Shampoos, Konditionierern, Körperreinigungsmitteln, Imprägnierlösungen für feuchte Reinigungstücher oder Hautreinigungsmitteln.

## Claims

1. Composition comprising water,
at least one biosurfactant selected from rhamnolipids and sophorolipids, and
at least one fatty acid selected from heptanoic acid, octanoic acid, pelargonic acid, decanoic acid, dodecanoic acid, tetradecanoic acid, palmitic acid, margaric acid, stearic acid, arachic acid, behenic acid, tetracosanoic acid, cerotic acid, triacontanoic acid, tuberculostearic acid, oleic acid, elaidic acid, erucic acid, linoleic acid, linolenic acid, elaeostearic acid, arachidonic acid, eicosapentaenoic acid and clupanodonic acid,
**characterized in that** the fraction of the sum of all surfactants in the composition is from 5 to 30% by weight, and that the fraction of fatty acid, based on the sum of fatty acid and surfactants, is from 0.1 to 20% by weight, **characterized in that** the composition comprises at least one thickener,
wherein at least 90% by weight of the thickeners are selected from xanthan gum, guar, agar agar, alginates, tyloses, cellulose, which are based in part or completely on renewable raw materials.

2. Composition according to Claim 1, **characterized in that** the fraction of thickeners in the composition is from 0.1 to 10% by weight, based on the composition.

3. Composition according to at least one of Claims 1 and 2, **characterized in that** the composition moreover comprises at least one surfactant which is not a biosurfactant.

4. Composition according to Claim 3, **characterized in that** the weight ratio of biosurfactants to surfactants which are not a biosurfactant is > 1:1.

5. Composition according to Claim 3, **characterized in that** the weight ratio of biosurfactants to surfactants which are not a biosurfactant is ≤ 1:1.

6. Composition according to one of Claims 1 to 5, **characterized in that** the composition comprises no surfactants which have sulphates or polyethylene glycol.

7. Composition according to one of Claims 1 to 6, **characterized in that** the composition has one or more natural dyes.

8. Composition according to one of Claims 1 to 7, **characterized in that** the composition is free from preservatives according to INCI.

9. Composition according to one of Claims 1 to 8, **characterized in that** the composition comprises propylene glycol, urea, glycerol, essential oils, phenylethyl alcohol and/or ethanol.

10. Composition according to one of Claims 1 to 9, **characterized in that** the composition has, as biosurfactants, mono- di- or polyrhamnolipids and/or sophorolipids.

11. Composition according to one of Claims 1 to 10, **characterized in that** the composition has perfume oils or fragrances, where the fraction of natural fragrances, based on the total number of fragrances in the perfume oils, is at least 50% by weight.

12. Composition according to one of Claims 1 to 11, **characterized in that** the composition is a body, skin or hair treatment composition.

13. Use of a composition according to one of Claims 1 to 12 as or for producing bath additives, shower gel, shampoos, conditioners, body cleansers, impregnation solutions for wet cleansing wipes or skin cleansers.

## Revendications

1. Composition contenant de l'eau,
au moins un biotensioactif choisi parmi les rhamnolipides et les sophorolipides, et
au moins un acide gras choisi parmi l'acide heptanoïque, l'acide octanoïque, l'acide pélargonique, l'acide décanoïque, l'acide dodécanoïque, l'acide tétradécanoïque, l'acide palmitique, l'acide margarique, l'acide stéarique, l'acide arachidique, l'acide béhénique, l'acide tétracosanoïque, l'acide cérotique, l'acide triacontanique, l'acide tuberculostéarique, l'acide oléique, l'acide élaïdique, l'acide érucique, l'acide linoléique, l'acide linolénique, l'acide éléostéarique, l'acide arachidonique, l'acide eicosapentaénoïque et l'acide clupanodonique,
**caractérisée en ce que** la proportion de la somme de tous les tensioactifs dans la composition est de 5 à 30 % en poids, et **en ce que** la proportion d'acide gras par rapport à la somme de l'acide gras et des tensioactifs est de 0,1 à 20 % en poids, **caractérisée en ce que** la composition contient au moins un épaississant,
au moins 90 % en poids des épaississants étant choisis parmi la gomme xanthane, gomme de guar, l'agar-agar, les alginates, les tyloses, la cellulose, qui sont partiellement ou entièrement à base de matières premières renouvelables.

2. Composition selon la revendication 1, **caractérisée en ce que** la proportion d'épaississants dans la composition est de 0,1 à 10 % en poids, par rapport à la composition.

3. Composition selon au moins l'une quelconque des revendications 1 ou 2, **caractérisée en ce que** la composition contient en outre au moins un tensioactif qui n'est pas un biotensioactif.

4. Composition selon la revendication 3, **caractérisée en ce que** le rapport en poids entre les biotensioactifs et les tensioactifs qui ne sont pas des biotensioactifs est > à 1 sur 1.

5. Composition selon la revendication 3, **caractérisée en ce que** le rapport en poids entre les biotensioactifs et les tensioactifs qui ne sont pas des biotensioactifs est ≤ à 1 sur 1.

6. Composition selon l'une quelconque des revendications 1 à 5, **caractérisée en ce que** la composition ne contient pas de tensioactifs qui comprennent des sulfates ou du polyéthylène glycol.

7. Composition selon l'une quelconque des revendications 1 à 6, **caractérisée en ce que** la composition comprend un ou plusieurs colorants naturels.

8. Composition selon l'une quelconque des revendications 1 à 7, **caractérisée en ce que** la composition est exempte de conservateurs selon l'INCI.

9. Composition selon l'une quelconque des revendications 1 à 8, **caractérisée en ce que** la composition contient du propylène glycol, de l'urée, de la glycérine, des huiles éthérées, de l'alcool phényléthylique et/ou de l'éthanol.

10. Composition selon l'une quelconque des revendications 1 à 9, **caractérisée en ce que** la composition comprend des mono-, di- ou polyrhamnolipides et/ou des sophorolipides en tant que biotensioactifs.

11. Composition selon l'une quelconque des revendications 1 à 10, **caractérisée en ce que** la composition comprend des huiles parfumées ou des parfums, la proportion de substances odorantes naturelles par rapport au nombre total de substances odorantes dans les huiles parfumées étant d'au moins 50 % en poids.

12. Composition selon l'une quelconque des revendications 1 à 11, **caractérisée en ce que** la composition est un produit de traitement du corps, de la peau ou des cheveux.

13. Utilisation d'une composition selon l'une quelconque des revendications 1 à 12 en tant que ou pour la fabrication d'additifs pour le bain, de gels douche, de shampoings, d'après-shampoings, de produits de nettoyage pour le corps, de solutions d'imprégnation pour lingettes humides ou de produits de nettoyage pour la peau.
